(19) Europäisches Patentamt
European Patent Office
Office européen des brevets

(11) **EP 4 516 770 A1**

(12) **EUROPEAN PATENT APPLICATION**
published in accordance with Art. 153(4) EPC

(43) Date of publication:
**05.03.2025 Bulletin 2025/10**

(21) Application number: **23796303.8**

(22) Date of filing: **24.04.2023**

(51) International Patent Classification (IPC):
***C07C 39/16*** (2006.01) ***C07C 37/20*** (2006.01)
***C07C 37/74*** (2006.01) ***C07C 37/84*** (2006.01)
***C07C 69/96*** (2006.01) ***C08G 64/30*** (2006.01)

(52) Cooperative Patent Classification (CPC):
(C-Sets available)
**C07C 37/0555; C07C 37/14; C07C 37/52; C07C 37/74; C07C 37/84; C08G 64/307** (Cont.)

(86) International application number:
**PCT/JP2023/016061**

(87) International publication number:
**WO 2023/210563 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.04.2022 JP 2022073594**

(71) Applicant: **Mitsubishi Chemical Corporation**
**Tokyo 100-8251 (JP)**

(72) Inventors:
- **NAKASHIMA, Yukie**
  **Tokyo 100-8251 (JP)**
- **UCHIYAMA, Kei**
  **Tokyo 100-8251 (JP)**

(74) Representative: **Vossius & Partner Patentanwälte Rechtsanwälte mbB**
**Siebertstrasse 3**
**81675 München (DE)**

(54) **COMPOSITION CONTAINING BISPHENOL, PRODUCTION METHOD FOR SAME, PRODUCTION METHOD FOR BISPHENOL A, AND PRODUCTION METHOD FOR POLYCARBONATE RESIN**

(57) Provided is a bisphenol (I)-containing composition that can give an improved recovery rate of isopropenylphenol when the composition is subjected to an alkaline degradation and recombination process. A bisphenol (I)-containing composition comprising: a bisphenol (I) comprising bisphenol A; and a compound (II) having a structure in which at least one hydroxyl group of a bisphenol is replaced with a carbonate ester group or a carbamoyloxy group, wherein the content of the bisphenol (I) in the bisphenol (I)-containing composition is 90% by mass or more; and the total number of moles of carbonyl bonds in the compound (II) relative to the mass of the bisphenol (I)-containing composition is 0.05 μmol/g or more.

EP 4 516 770 A1

(52) Cooperative Patent Classification (CPC): (Cont.)

C-Sets
**C07C 37/0555, C07C 39/16;**
**C07C 37/14, C07C 39/16;**
**C07C 37/52, C07C 39/04;**
**C07C 37/52, C07C 39/06;**
**C07C 37/74, C07C 39/04;**
**C07C 37/74, C07C 39/06;**
**C07C 37/84, C07C 39/16**

## Description

### Technical Field

The present invention relates to a bisphenol-containing composition and a method for producing the same. The present invention also relates to a method for producing bisphenol A and a method for producing a polycarbonate resin.

### Background Art

Bisphenols are useful as starting materials for polymeric materials such as a polycarbonate resin, an epoxy resin and an aromatic polyester resin. Representative examples of the known bisphenols include 2,2-bis(4-hydroxyphenyl) propane and 2,2-bis(4-hydroxy-3-methylphenyl)propane (Patent Literatures 1 and 2).

### Citation List

#### Patent Literature

Patent Literature 1: Japanese Patent Laid-Open No. 62-138443
Patent Literature 2: Japanese Patent Laid-Open No. 2014-40376
Patent Literature 3: Japanese Patent Publication No. 49-48319
Patent Literature 4: Japanese Patent Laid-Open No. 1-230538

### Summary of Invention

#### Technical Problem

A polycarbonate resin, which is a representative use of bisphenol, is required to be colorless and transparent. The color tone of the polycarbonate resin is greatly affected by the color tone of starting materials therefor. Therefore, the color tone of the starting material, bisphenol, is also required to be colorless. However, bisphenol is easily affected by impurities in starting materials therefor and deterioration of its production equipment therefor and it easily becomes colored, so that the quality is not easy to control.

Examples of the method for purifying bisphenol include recrystallization, distillation and column purification.

For bisphenol A among bisphenols, an alkaline degradation and recombination process is known, in which phenol and isopropenylphenol obtained by degradation under a basic catalyst are distilled and purified, and then recombined under an acidic catalyst to produce bisphenol A (for example, Patent Literatures 3 and 4).

The alkaline degradation and recombination process for bisphenol A includes an alkaline degradation step of degrading bisphenol A into phenol and isopropenylphenol under a basic catalyst; a distillation step of distilling the produced phenol and isopropenylphenol to purify them; and a recombination reaction step of recombining the purified phenol and isopropenylphenol under an acidic catalyst to synthesize bisphenol A. This alkaline degradation and recombination process is advantageous in that bisphenol A, which has a high boiling point, can be purified through conversion it into compounds having a low boiling point and distillation thereof, thereby making it easy to separate it from color-causing substances.

However, in the alkaline degradation step of the alkaline degradation and recombination process for bisphenol A, bisphenol A is degraded into phenol and isopropenylphenol in the presence of a basic catalyst under high temperature conditions, and undesired reactions therefore easily occur. In particular, isopropenylphenol easily self-condenses due to its high reactivity, proposing a problem with the recovery rate.

The present invention has been made in consideration of the above-described conventional situations. An object of the present invention is to provide a bisphenol (I)-containing composition that can give an improved recovery rate of isopropenylphenol when the composition is subjected to an alkaline degradation and recombination process, and a method for producing the same.

Another object of the present invention is to provide a method for producing bisphenol A by using the above-described bisphenol (I)-containing composition to efficiently obtain bisphenol A, and a method for producing a polycarbonate resin by using the bisphenol A as a starting material.

Solution to Problem

After diligent research for solving the above problems, the present inventors have found that isopropenylphenol can be efficiently obtained by using, as a starting material, a bisphenol (I)-containing composition that contains bisphenol A and a specific amount of a compound (II) having a specific structure and subjecting it to an alkaline degradation step of an alkaline degradation and recombination process. They have found that the yield of bisphenol A in a recombination reaction step can be thus improved. They further have found a method for producing a polycarbonate resin by using the obtained bisphenol A.

Specifically, the present invention is as follows.

<1> A bisphenol (I)-containing composition comprising: a bisphenol (I) comprising bisphenol A; and a compound (II) having a structure in which at least one hydroxyl group of a bisphenol is replaced with a carbonate ester group or a carbamoyloxy group; wherein the content of the bisphenol (I) in the bisphenol (I)-containing composition is 90% by mass or more; and the total number of moles of carbonyl bonds in the compound (II) relative to the mass of the bisphenol (I)-containing composition is 0.05 μmol/g or more.

<2> The bisphenol (I)-containing composition according to the above <1>, wherein the total number of moles of carbonyl bonds in the compound (II) relative to the mass of the bisphenol (I)-containing composition is 50 μmol/g or less.

<3> The bisphenol (I)-containing composition according to the above <1> or <2>, wherein the compound (II) is a compound represented by the following formula (2) and/or formula (3):

[Chemical Formula 1]

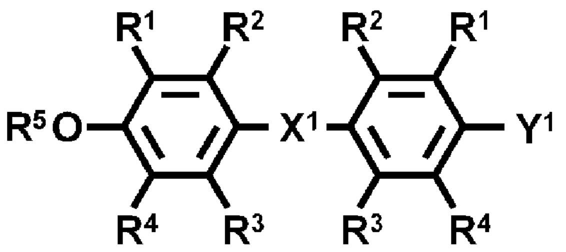

(2)

wherein

$R^1$ to $R^4$ are each independently a hydrogen atom, a halogen atom, an alkyl group, an aryl group or an alkoxy group;

$X^1$ is $*-CR^{10}R^{11}-*$ (wherein * represents the bonding position to the benzene ring, and $R^{10}$ and $R^{11}$ are each independently a hydrogen atom, a halogen atom, an alkyl group, an aryl group or an alkoxy group), a cycloalkylidene group, a fluorenylidene group, a xanthenylidene group or a thioxanthenylidene group;

$R^5$ is a hydrogen atom, an alkyl group or an aryl group; and

$Y^1$ is a carbonate ester group or a carbamoyloxy group;

[Chemical Formula 2]

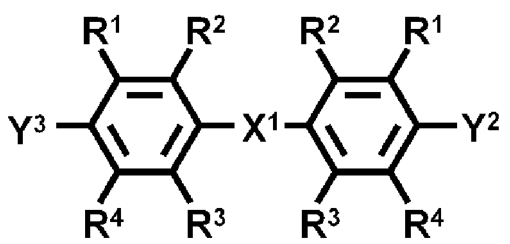

(3)

wherein

$R^1$ to $R^4$ and $X^1$ have the same meanings as defined for $R^1$ to $R^4$ and $X^1$ in the formula (2); and

$Y^2$ and $Y^3$ are each independently a carbonate ester group or a carbamoyloxy group.

<4> The bisphenol (I)-containing composition according to any of the above <1> to <3>, wherein the carbonate ester

group is a group represented by the following formula (G1) or formula (G2):

[Chemical Formula 3]

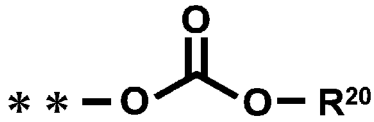

(G 1)

wherein

** represents the bonding position to the benzene ring of the bisphenol skeleton; and
$R^{20}$ is a hydrogen atom, an alkyl group or an aryl group;

[Chemical Formula 4]

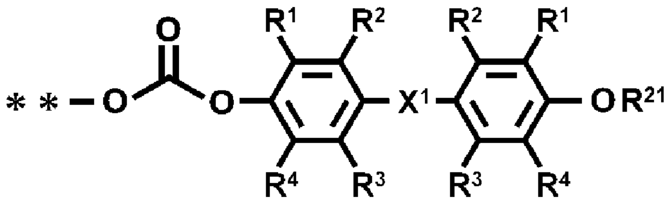

(G 2)

wherein
** represents the bonding position to the benzene ring of the bisphenol skeleton;
$R^1$ to $R^4$ are each independently a hydrogen atom, a halogen atom, an alkyl group, an aryl group or an alkoxy group;
$X^1$ is *-$CR^{10}R^{11}$-* (wherein * represents the bonding position to the benzene ring, and $R^{10}$ and $R^{11}$ are each independently a hydrogen atom, a halogen atom, an alkyl group, an aryl group or an alkoxy group), a cycloalkylidene group, a fluorenylidene group, a xanthenylidene group or a thioxanthenylidene group; and
$R^{21}$ is a hydrogen atom, an alkyl group or an aryl group.

<5> The bisphenol (I)-containing composition according to any of the above <1> to <4>, wherein the content of bisphenol A in the bisphenol (I) is 90% by mass or more.
<6> The bisphenol (I)-containing composition according to any of the above <1> to <5>, wherein the compound (II) is one or more selected from the group consisting of compounds represented by the following formulas (i) to (v).

[Chemical Formula 5]

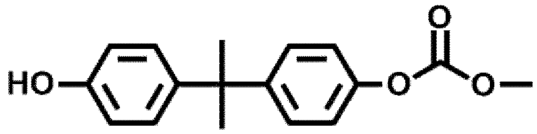

( i )

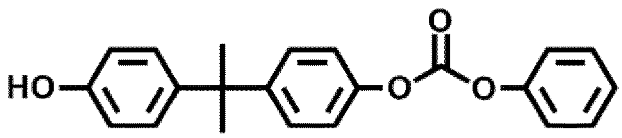

( ii )

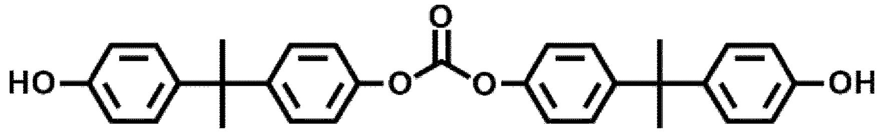

( iii )

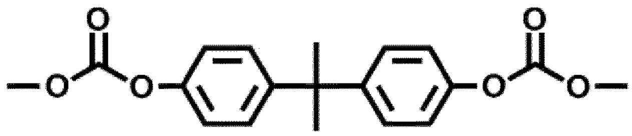

( iv )

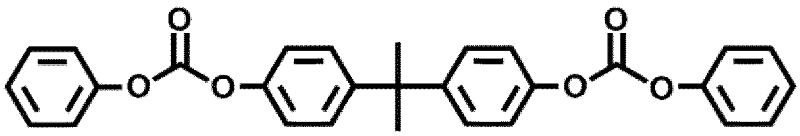

( v )

<7> A method for producing the bisphenol (I)-containing composition according to any of the above <1> to <6>, comprising a BPA production step of producing the compound (II) as a by-product when producing bisphenol A.

<8> The method for producing a bisphenol (I)-containing composition according to the above <7>, wherein the BPA production step comprises degrading a polycarbonate resin to produce bisphenol A while producing the compound (II) as a by-product, thereby obtaining the bisphenol (I)-containing composition comprising bisphenol A and the compound (II).

<9> A method for producing the bisphenol (I)-containing composition according to any of the above <1> to <6>, comprising a step of adding the compound (II) to the bisphenol (I) comprising bisphenol A.

<10> A method for producing bisphenol A, comprising: an alkaline degradation step of degrading the bisphenol (I)-containing composition according to any of the above <1> to <6> in the presence of a basic catalyst to obtain a degradation liquid containing isopropenylphenol and phenol; a distillation step of distilling the degradation liquid to obtain a distillate containing isopropenylphenol and phenol; and a recombination reaction step of recombining isopropenylphenol and phenol by contacting the obtained distillate with an acidic catalyst to produce bisphenol A, thereby obtaining a solution containing bisphenol A.

<11> The method for producing bisphenol A according to the above <10>, comprising: a step of feeding the solution containing bisphenol A obtained in the recombination reaction step to a BPA synthesis means and/or a concentration means of a BPA production plant, wherein the BPA production plant comprises: the BPA synthesis means of performing dehydrative condensation of acetone and phenol in the presence of an acidic catalyst to obtain a reaction liquid c1 containing bisphenol A; the concentration means of distilling off an unreacted acetone and water from the reaction liquid c1 to obtain a concentrate c2; a separation means of subjecting the concentrate c2 to crystallization to

obtain a slurry liquid and subjecting the slurry liquid to solid-liquid separation to separate it into a mother liquid c3 and a cake containing a crystal of a phenol adduct of bisphenol A; a BPA purification means of purifying the cake to obtain bisphenol A; a first circulation means of circulating a portion of the mother liquid c3 and feeding it to the BPA synthesis means and/or the concentration means; a mother liquid purification means of carrying out distillation while or after degrading bisphenol A contained in the mother liquid c3 to obtain a distillate c4 containing isopropenylphenol and phenol, and contacting the obtained distillate c4 with an acidic catalyst to obtain a reaction liquid c5 containing bisphenol A; and a second circulation means of feeding the reaction liquid c5 to the BPA synthesis means and/or the concentration means.

<12> The method for producing bisphenol A according to the above <10>, wherein: the bisphenol (I)-containing composition is fed to a mother liquid purification means of a BPA production plant, wherein the BPA production plant comprises: a BPA synthesis means of performing dehydrative condensation of acetone and phenol in the presence of an acidic catalyst to obtain a reaction liquid c1 containing bisphenol A; a concentration means of distilling off an unreacted acetone and water from the reaction liquid c1 to obtain a concentrate c2; a separation means of subjecting the concentrate c2 to crystallization to obtain a slurry liquid and subjecting the slurry liquid to solid-liquid separation to separate it into a mother liquid c3 and a cake containing a crystal of a phenol adduct of bisphenol A; a BPA purification means of purifying the cake to obtain bisphenol A; a first circulation means of circulating a portion of the mother liquid c3 and feeding it to the BPA synthesis means and/or the concentration means; the mother liquid purification means of carrying out distillation while or after degrading bisphenol A contained in the mother liquid c3 to obtain a distillate c4 containing isopropenylphenol and phenol, and contacting the obtained distillate c4 with an acidic catalyst to obtain a reaction liquid c5 containing bisphenol A; and a second circulation means of feeding the reaction liquid c5 to the BPA synthesis means and/or the concentration means; and in the mother liquid purification means, the alkaline degradation step, the distillation step and the recombination reaction step are carried out.

<13> The method for producing bisphenol A according to the above <10>, wherein: the distillate obtained in the distillation step is fed to a mother liquid purification means of a BPA production plant, wherein the BPA production plant comprises: a BPA synthesis means of performing dehydrative condensation of acetone and phenol in the presence of an acidic catalyst to obtain a reaction liquid c1 containing bisphenol A; a concentration means of distilling off an unreacted acetone and water from the reaction liquid c1 to obtain a concentrate c2; a separation means of subjecting the concentrate c2 to crystallization to obtain a slurry liquid and subjecting the slurry liquid to solid-liquid separation to separate it into a mother liquid c3 and a cake containing a crystal of a phenol adduct of bisphenol A; a BPA purification means of purifying the cake to obtain bisphenol A; a first circulation means of circulating a portion of the mother liquid c3 and feeding it to the BPA synthesis means and/or the concentration means; the mother liquid purification means of carrying out distillation while or after degrading bisphenol A contained in the mother liquid c3 to obtain a distillate c4 containing isopropenylphenol and phenol, and contacting the obtained distillate c4 with an acidic catalyst to obtain a reaction liquid c5 containing bisphenol A; and a second circulation means of feeding the reaction liquid c5 to the BPA synthesis means and/or the concentration means; and in the mother liquid purification means, the recombination reaction step is carried out.

<14> A method for producing a polycarbonate resin comprising a first step of obtaining bisphenol A by the method for producing bisphenol A according to any of the above <10> to <13>; and a second step of polymerizing bisphenol A obtained in the first step to obtain a polycarbonate resin.

<15> The method for producing a polycarbonate resin according to the above <14>, wherein the viscosity average molecular weight of the polycarbonate resin is 15,000 or more and 35,000 or less.

Advantageous Effects of Invention

According to the present invention, provided are a bisphenol (I)-containing composition that can give an improved recovery rate of isopropenylphenol when subjecting the composition to an alkaline degradation and recombination process, and a method for producing the same.

In addition, provided are a method for producing bisphenol A by using the above-described bisphenol (I)-containing composition to efficiently obtain bisphenol A, and a method for producing a polycarbonate resin by using the bisphenol A as a starting material.

Brief Description of Drawings

[Figure 1] Figure 1 is a diagram illustrating one example of a method for producing bisphenol A according to the present invention.

[Figure 2] Figure 2 is a diagram illustrating one example of a method for producing bisphenol A according to the

present invention.

Description of Embodiments

Hereinafter, modes for carrying out the present invention will be described in detail. The description of constituent elements below is for one example (representative example) for embodiments of the present invention, and the present invention is not limited to the following contents unless the gist thereof is changed. When the term "to" is used herein for numerical values or values of physical properties, it intends to include the numerical values or values of physical properties before and after the term "to".

[Bisphenol (I)-containing composition]

The bisphenol (I)-containing composition of the present invention relates to a bisphenol (I)-containing composition comprising: a bisphenol (I) comprising bisphenol A; and a compound (II) having a structure in which at least one hydroxyl group of a bisphenol is replaced with a carbonate ester group or a carbamoyloxy group; wherein the content of the bisphenol (I) in the bisphenol (I)-containing composition is 90% by mass or more; and the total number of moles of carbonyl bonds in the compound (II) relative to the mass of the bisphenol (I)-containing composition is 0.05 μmol/g or more (hereinafter sometimes referred to as "bisphenol (I)-containing composition of the present invention").

The bisphenol (I)-containing composition contains: 90% by mass or more of the bisphenol (I) comprising bisphenol A; and a specific amount of the compound (II) having a structure in which at least one hydroxyl group of a bisphenol is replaced with a carbonate ester group or a carbamoyloxy group, and is thus excellent in the recovery rate of isopropenylphenol when the composition is subjected to an alkaline degradation and recombination process. In addition, bisphenol A can be efficiently obtained by using the recovered isopropenylphenol as a starting material, and a method for producing a polycarbonate resin by using the obtained bisphenol A as a starting material can be provided.

<Bisphenol (I)>

The bisphenol (I)-containing composition contains, as a component, a bisphenol (I) comprising bisphenol A. The bisphenol (I) is usually a bisphenol compound represented by the following formula (1):

[Chemical Formula 6]

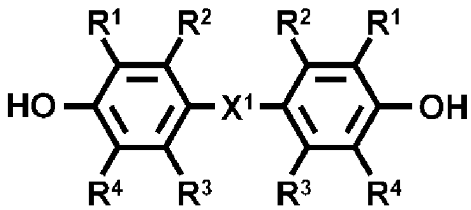

(1)

In the formula (1), $R^1$ to $R^4$ are each independently a hydrogen atom, a halogen atom, an alkyl group, an aryl group or an alkoxy group. The alkyl group, aryl group and alkoxy group may be either substituted or unsubstituted. The number of carbon atoms of each of the substituted or unsubstituted alkyl group and the substituted or unsubstituted alkoxy group is 1 or more, and for the upper limit thereof, the number of carbon atoms is preferably 12 or less, more preferably 8 or less and even more preferably 6 or less. The number of carbon atoms of the substituted or unsubstituted aryl group is 6 or more, and for the upper limit thereof, the number of carbon atoms is preferably 12 or less and more preferably 8 or less.

Examples of $R^1$ to $R^4$ include a hydrogen atom, a fluoro group, a chloro group, a bromo group, an iodo group, a methyl group, an ethyl group, an n-propyl group, an i-propyl group, an n-butyl group, an i-butyl group, a t-butyl group, an n-pentyl group, an i-pentyl group, an n-hexyl group, an n-heptyl group, an n-octyl group, an n-nonyl group, an n-decyl group, an n-undecyl group, an n-dodecyl group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group, a 2,6-dimethylphenyl group, a methoxy group, an ethoxy group, an n-propoxy group, an i-propoxy group, an n-butoxy group, an i-butoxy group, a t-butoxy group, an n-pentyloxy group, an i-pentyloxy group, an n-hexyloxy group, an n-heptyloxy group, an n-octyloxy group, an n-nonyloxy group, an n-decyloxy group, an n-undecyloxy group and an n-dodecyloxy group.

In the formula (1), $X^1$ is *-$CR^{10}R^{11}$-*, a cycloalkylidene group, a fluorenylidene group, a xanthenylidene group or a thioxanthenylidene group.

In "*-$CR^{10}R^{11}$-*", * represents the bonding position to the benzene ring. $R^{10}$ and $R^{11}$ are each independently a hydrogen atom, a halogen atom, an alkyl group, an aryl group or an alkoxy group. The alkyl group, aryl group and alkoxy group and alkoxy

group may be either substituted or unsubstituted. The number of carbon atoms of each of the substituted or unsubstituted alkyl group and the substituted or unsubstituted alkoxy group is 1 or more, and for the upper limit thereof, the number of carbon atoms is preferably 12 or less, more preferably 8 or less and even more preferably 6 or less. The number of carbon atoms of the substituted or unsubstituted aryl group is 6 or more, and for the upper limit thereof, the number of carbon atoms is preferably 12 or less and more preferably 8 or less.

Examples of $R^{10}$ and $R^{11}$ include a hydrogen atom, a fluoro group, a chloro group, a bromo group, an iodo group, a methyl group, an ethyl group, an n-propyl group, an i-propyl group, an n-butyl group, an i-butyl group, a t-butyl group, an n-pentyl group, an i-pentyl group, an n-hexyl group, an n-heptyl group, an n-octyl group, an 2-ethylhexyl group, an n-nonyl group, an n-decyl group, an n-undecyl group, an n-dodecyl group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group, a 2,6-dimethylphenyl group, a methoxy group, an ethoxy group, an n-propoxy group, an i-propoxy group, an n-butoxy group, an i-butoxy group, a t-butoxy group, an n-pentyloxy group, an i-pentyloxy group, an n-hexyloxy group, an n-heptyloxy group, an n-octyloxy group, an n-nonyloxy group, an n-decyloxy group, an n-undecyloxy group and an n-dodecyloxy group.

The cycloalkylidene group is a divalent group obtained by removing the two hydrogen atoms bonded to one carbon atom of a cycloalkane. The cycloalkylidene group may be either substituted or unsubstituted, and may contain a hetero atom in the ring structure. The number of carbon atoms in the substituted or unsubstituted cycloalkylidene group is preferably 3 or more and 12 or less.

Examples of the cycloalkyliden group include cyclopropylidene, cyclobutylidene, cyclopentylidene, cyclohexylidene, 3,3,5-trimethylcyclohexylidene, cycloheptylidene, cyclooctylidene, cyclononylidene, cyclodecylidene, cycloundecylidene and cyclododecylidene.

The fluorenylidene group (fluorene-9,9-diyl group) is a divalent group obtained by removing the two hydrogen atoms bonded to the carbon atom at position 9 of fluorene. The xanthenylidene group (xanthene-9,9-diyl group) is a divalent group obtained by removing the two hydrogen atoms bonded to the carbon atom at position 9 of xanthene. The thioxanthenylidene group (thioxanthene-9,9-diyl group) is a divalent group obtained by removing the two hydrogen atoms bonded to the carbon atom at position 9 of thioxanthene.

<Bisphenol A>

The bisphenol (I) contained in the bisphenol (I)-containing composition of the present invention contains bisphenol A as a main component. Bisphenol A refers to 2,2-bis(4-hydroxyphenyl)propane, that is, a compound represented by the above formula (1) wherein $R^1$ to $R^4$ are hydrogen atoms, and $X^1$ is "*-$CR^{10}R^{11}$-*" wherein $R^{10}$ and $R^{11}$ are methyl groups. Hereinafter, bisphenol A is sometimes referred to as "BPA".

<Other bisphenols>

The bisphenol (I) may consist only of bisphenol A, but may contain a bisphenol other than bisphenol A, in addition to bisphenol A.

Examples of the other bisphenol include the compounds represented by the above formula (1), other than bisphenol A. Specifically, examples thereof include the following bisphenol (1a) and/or bisphenol (1b).

Bisphenol (1a): a compound represented by the above formula (1) wherein $R^1$ to $R^4$ are each independently a hydrogen atom, a halogen atom, an alkyl group, an aryl group or an alkoxy group, and $X^1$ is *-$CR^{10}R^{11}$-* (wherein * represents the bonding position to the benzene ring, $R^{10}$ is a hydrogen atom, a halogen atom, an alkyl group, an aryl group or an alkoxy group, and $R^{11}$ is a hydrogen atom, a halogen atom, an alkyl group having 2 or more carbon atoms, an aryl group or an alkoxy group), a cycloalkylidene group or a fluorenylidene group.

Bisphenol (1b): a compound represented by the above formula (1) wherein $R^1$ to $R^4$ are each independently a hydrogen atom, a halogen atom, an alkyl group, an aryl group or an alkoxy group and at least one of $R^1$ to $R^4$ is a halogen atom, an alkyl group, an aryl group or an alkoxy group, and $X^1$ is *-$CR^{10}R^{11}$-* (wherein * represents the bonding position to the benzene ring, $R^{10}$ and $R^{11}$ are each independently a hydrogen atom, a halogen atom, an alkyl group, an aryl group or an alkoxy group), a cycloalkylidene group or a fluorenylidene group.

In the bisphenol (1a) represented by the above formula (1), $R^2$ and $R^3$ are each preferably a hydrogen atom, which is not sterically bulky. It is more preferable that $R^1$ and $R^4$ should be each independently a hydrogen atom or an alkyl group, and that $R^2$ and $R^3$ should be each a hydrogen atom.

In the bisphenol (1b) represented by the above formula (1), $R^2$ and $R^3$ are each preferably a hydrogen atom, which is not sterically bulky. It is more preferable that $R^1$ should be an alkyl group, and that $R^4$ should be a hydrogen atom or an alkyl group, and that $R^2$ and $R^3$ should be each a hydrogen atom.

Specific examples of the other bisphenol represented by the above formula (1) include, but not limited to, 2,2-bis(4-hydroxy-3-methylphenyl)propane, 2,2-bis(4-hydroxy-3,5-dimethylphenyl)propane, 1,1-bis(4-hydroxy-3-methylphenyl)cyclohexane, 9,9-bis(4-hydroxy-3-methylphenyl)fluorene, 3,3-bis(4-hydroxyphenyl)pentane, 3,3-bis(4-hydroxy-3-methylphenyl)pentane, 2,2-bis(4-hydroxyphenyl)pentane, 2,2-bis(4-hydroxy-3-methylphenyl) pentane, 3,3-bis(4-hydroxyphenyl)heptane, 3,3-bis(4-hydroxy-3-methylphenyl)heptane, 2,2-bis(4-hydroxyphenyl) heptane, 2,2-bis(4-hydroxy-3-methylphenyl) heptane, 4,4-bis(4-hydroxyphenyl)heptane and 4,4-bis(4-hydroxy-3-methylphenyl)heptane.

Among them, the other bisphenol contained in the bisphenol (I)-containing composition of the present invention is preferably any one selected from the group consisting of 2,2-bis(4-hydroxy-3-methylphenyl)propane, 2,2-bis(4-hydroxy-3,5-dimethylphenyl)propane and 1,1-bis(4-hydroxy-3-methylphenyl)cyclohexane, and more preferably 2,2-bis(4-hydroxy-3-methylphenyl)propane.

The bisphenol (I)-containing composition of the present invention contains 90% by mass or more of the bisphenol (I). The content of the bisphenol (I) (the mass of the bisphenol (I)/the mass of the bisphenol (I)-containing composition × 100 (%)) is preferably 95% by mass or more, more preferably 98% by mass or more, and even more preferably 99% by mass or more. A content of the bisphenol (I) less than the above lower limit is not preferable for using it as a bisphenol. For the upper limit, the content of the bisphenol (I) in the bisphenol (I)-containing composition of the present invention is about 99.998% by mass or less or about 99.990% by mass or less, in view of ensuring the content of the compound (II), the production cost, adjustment of the substance-amount ratio thereof to diphenyl carbonate in the reaction for producing a polycarbonate resin, and mechanical properties, such as surface hardness and brittleness, of a polycarbonate resin.

The bisphenol (I) contained in the bisphenol (I)-containing composition of the present invention preferably has a bisphenol A content of 90% by mass or more. The content of bisphenol A in the bisphenol (I) (the mass of the bisphenol A/the mass of the bisphenol (I) × 100 (%)) is preferably 95% by mass or more, more preferably 98% by mass or more, and even more preferably 99% by mass or more. The bisphenol (I) may consist of bisphenol A. Such a content of bisphenol A is preferable because it allows bisphenol A to be produced more efficiently in the method for producing bisphenol A, which will be described later.

The bisphenol (I) can be detected and quantified using a standard reversed-phase column for high-speed analysis. Examples of the standard reversed-phase column for high-speed analysis include an ODS-based column having an inner diameter of 3 to 4.6 mm, a length of 10 to 30 cm and a particle size of 3 to 5 μm. Specifically, Scherzo SM-C18, Cadenza CD-C18 and Unison UK-C18 manufactured by Imtakt Corporation can be used. The sizes thereof will be described in detail in the Examples.

<Compound (II)>

The compound (II) contained in the bisphenol (I)-containing composition of the present invention is a compound having a structure in which at least one hydroxyl group of a bisphenol is replaced with a carbonate ester group or a carbamoyloxy group.

The carbonate ester group is a functional group having "-O-C(=O)-O-". The carbonate ester group may have a number of carbonate ester bonds.

The carbonate ester group is preferably represented by the following formula (G1) or formula (G2).

[Chemical Formula 7]

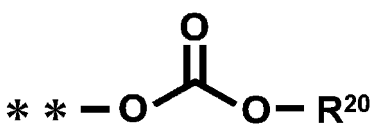

(G 1)

In the formula (G1), ** represents the bonding position to the benzene ring of the bisphenol skeleton. $R^{20}$ is a hydrogen atom, an alkyl group or an aryl group, and preferably an alkyl group or an aryl group. The alkyl group and aryl group may be either substituted or unsubstituted. The number of carbon atoms of the substituted or unsubstituted alkyl group is 1 or more, and preferably 12 or less, more preferably 8 or less and even more preferably 6 or less. The number of carbon atoms of the substituted or unsubstituted aryl group is 6 or more, and preferably 12 or less and more preferably 8 or less.

[Chemical Formula 8]

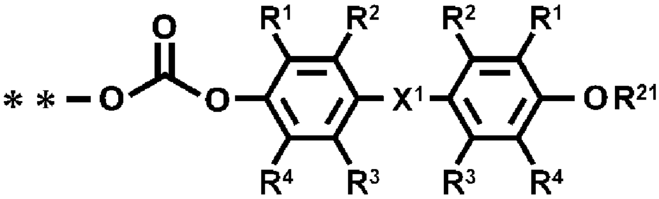

(G2)

In the formula (G2), ** represents the bonding position to the benzene ring of the bisphenol skeleton. $R^1$ to $R^4$ and $X^1$ have the same meanings as defined for $R^1$ to $R^4$ and $X^1$ in the formula (1), and preferred examples and specific examples are also as described for the formula (1).

In the formula (G2), $R^{21}$ is a hydrogen atom, an alkyl group or an aryl group. The alkyl group and aryl group may be either substituted or unsubstituted. The number of carbon atoms of the substituted or unsubstituted alkyl group is 1 or more, and preferably 12 or less, more preferably 8 or less and even more preferably 6 or less. The number of carbon atoms of the substituted or unsubstituted aryl group is 6 or more, and preferably 12 or less and more preferably 8 or less.

Examples of the compound (II) having the carbonate ester group represented by the formula (G2) include a bisphenol dimer.

The carbamoyloxy group is a functional group having "-O-C(=O)-N<".

The carbamoyloxy group is preferably represented by the following formula (G3).

[Chemical Formula 9]

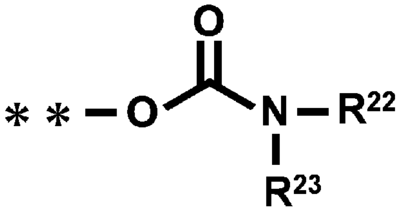

(G3)

In the formula (G3), ** represents the bonding position to the benzene ring of the bisphenol skeleton. $R^{22}$ and $R^{23}$ are each independently a hydrogen atom, an alkyl group or an aryl group. The alkyl group and aryl group may be either substituted or unsubstituted. The number of carbon atoms of the substituted or unsubstituted alkyl group is 1 or more, and preferably 12 or less, more preferably 8 or less and even more preferably 6 or less. The number of carbon atoms of the substituted or unsubstituted aryl group is 6 or more, and preferably 12 or less and more preferably 8 or less.

Examples of $R^{20}$ to $R^{23}$ include a hydrogen atom, a methyl group, an ethyl group, an n-propyl group, an i-propyl group, an n-butyl group, an i-butyl group, a t-butyl group, an n-pentyl group, an i-pentyl group, an n-hexyl group, an n-heptyl group, an n-octyl group, an 2-ethylhexyl group, an n-nonyl group, an n-decyl group, an n-undecyl group, an n-dodecyl group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group and a 2,6-dimethylphenyl group.

The compound (II) preferably includes a compound represented by the following formula (2) and/or formula (3), and more preferably, the compound (II) is a compound represented by the following formula (2) and/or formula (3).

[Chemical Formula 10]

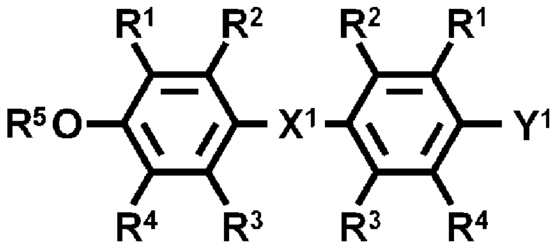

(2)

In the formula (2), $R^1$ to $R^4$ and $X^1$ have the same meanings as defined for $R^1$ to $R^4$ and $X^1$ in the formula (1), and preferred examples and specific examples are also as described for the formula (1). $R^1$ to $R^4$ and $X^1$ in the formula (2) may

be the same as or different from $R^1$ to $R^4$ and $X^1$ in the above formula (1), respectively, and they are preferably the same.

In the formula (2), $R^5$ is a hydrogen atom, an alkyl group or an aryl group. $R^5$ is preferably a hydrogen atom or an alkyl group, and more preferably a hydrogen atom. The alkyl group and aryl group may be either substituted or unsubstituted. The number of carbon atoms of the substituted or unsubstituted alkyl group is 1 or more, and for the upper limit thereof, the number of carbon atoms is preferably 12 or less, more preferably 8 or less and even more preferably 6 or less. The number of carbon atoms of the substituted or unsubstituted aryl group is 6 or more, and for the upper limit thereof, number of carbon atoms is preferably 12 or less and more preferably 8 or less.

Examples of $R^5$ include a hydrogen atom, a methyl group, an ethyl group, an n-propyl group, an i-propyl group, an n-butyl group, an i-butyl group, a t-butyl group, an n-pentyl group, an i-pentyl group, an n-hexyl group, an n-heptyl group, an n-octyl group, an 2-ethylhexyl group, an n-nonyl group, an n-decyl group, an n-undecyl group, an n-dodecyl group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group and a 2,6-dimethylphenyl group.

In the formula (2), $Y^1$ is a carbonate ester group or a carbamoyloxy group. $Y^1$ can be a carbonate ester group represented by the above formula (G1) or formula (G2) and a carbamoyloxy group represented by the above formula (G3). $Y^1$ is preferably a carbonate ester group, preferably a carbonate ester group having 2 to 30 carbon atoms, more preferably a carbonate group ester having 2 to 25 carbon atoms, and even more preferably a carbonate ester group having 2 to 20 carbon atoms.

[Chemical Formula 11]

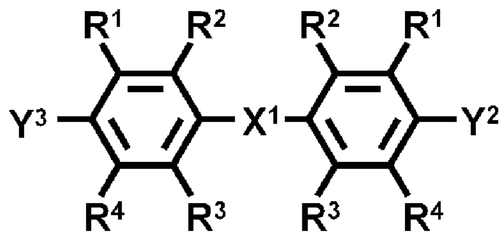

(3)

$R^1$ to $R^4$ and $X^1$ in the formula (3) have the same meanings as defined for $R^1$ to $R^4$ and $X^1$ in the formula (1) and (2). Preferred examples and specific examples thereof are as described for the above formula (1). $R^1$ to $R^4$ and $X^1$ in the formula (3) may be the same as or different from $R^1$ to $R^4$ and $X^1$ in the above formula (1) and (2), respectively, and they are preferably the same.

In the formula (3), $Y^2$ and $Y^3$ are each independently a carbonate ester group or a carbamoyloxy group. $Y^2$ and $Y^3$ each independently can be a carbonate ester group represented by the above formula (G1) or formula (G2) or a carbamoyloxy group represented by the above formula (G3). In the formula (3), $Y^2$ and $Y^3$ are each independently preferably a carbonate ester group, more preferably a carbonate ester group having 2 to 13 carbon atoms and even more preferably a carbonate ester group having 2 to 9 carbon atoms.

In the formula (3), $Y^2$ and $Y^3$ are preferably the same groups, and $Y^2$ and $Y^3$ are more preferably the same carbonate ester groups.

When the bisphenol (I)-containing composition of the present invention contains a compound (II) represented by the formula (2), the compound (II) represented by the formula (2) may be more than one type. When the bisphenol (I)-containing composition contains a compound (II) represented by the formula (3), the compound (II) represented by the formula (3) may be more than one type.

The compound (II) preferably includes a compound having a structure in which at least one hydroxyl group of the bisphenol A is replaced with a carbonate ester group or a carbamoyloxy group, because it functions as a starting material for isopropenylphenol when subjected to an alkaline degradation and recombination process.

When the bisphenol (I)-containing composition of the present invention contains a compound (II) represented by the formula (2), the compound (II) contained is preferably represented by the formula (2) wherein $R^1$ to $R^4$ are hydrogen atoms, $X^1$ is "*-$CR^{10}R^{11}$-*" wherein $R^{10}$ and $R^{11}$ are methyl groups, $R^5$ is a hydrogen atom, an alkyl group or an aryl group, and $Y^1$ is a carbonate ester group or a carbamoyloxy group.

When the bisphenol (I)-containing composition of the present invention contains a compound (II) represented by the formula (3), the compound (II) contained is preferably represented by the formula (3) wherein $R^1$ to $R^4$ are hydrogen atoms, $X^1$ is "*-$CR^{10}R^{11}$-*" wherein $R^{10}$ and $R^{11}$ are methyl groups, $Y^2$ and $Y^3$ are each independently a carbonate ester group or a carbamoyloxy group.

Among them, the compound (II) preferably includes one or more selected from the group consisting of the compounds represented by the following formulas (i) to (v), and more preferably, the compound (II) is one or more selected from the group consisting of the compounds represented by the following formulas (i) to (v).

[Chemical Formula 12]

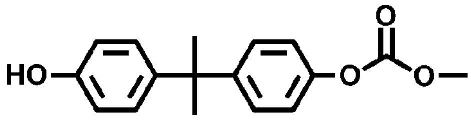

( i )

[Chemical Formula 13]

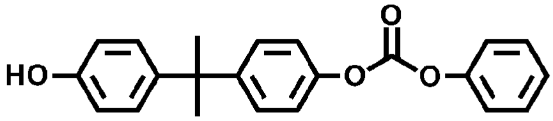

( ii )

[Chemical Formula 14]

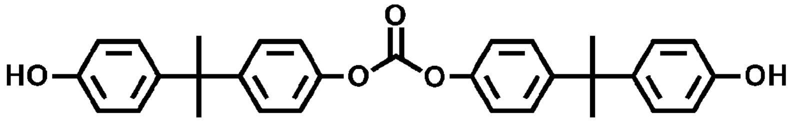

( iii )

[Chemical Formula 15]

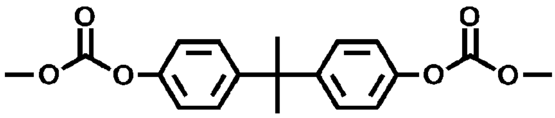

( iv )

[Chemical Formula 16]

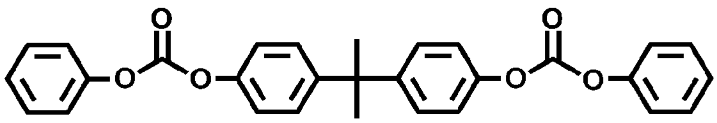

( v )

If the molecular weight of the compound (II) is too high, the viscosity of the bisphenol (I)-containing composition increases. Therefore, the molecular weight is preferably 300 to 1,000, and may be 300 to 800, 300 to 650, or the like.

The total number of moles of carbonyl bonds (-C(=O)-) in the compound (II) relative to the mass of the bisphenol (I)-containing composition of the present invention is 0.05 μmol/g or more. It is preferably 0.06 μmol/g or more, and may be 0.08 μmol/g or more, or 0.10 μmol/g or more. For the upper limit thereof, it may be 50 μmol/g or less, 45 μmol/g or less, 40 μmol/g or less, 30 μmol/g or less, or 25 μmol/g or less, and a smaller upper limit is more preferable in the listed order, in view of workability. It may be 20 μmol/g or less, 15 μmol/g or less, 10 μmol/g or less, 5 μmol/g or less, or 2.5 μmol/g or less, for example.

If the total number of moles of carbonyl bonds in the compound (II) relative to the mass of the bisphenol (I)-containing composition is less than the above lower limit, the content of the compound (II) is low, and it is therefore difficult to obtain a bisphenol (I)-containing composition excellent in the recovery rate of isopropenylphenol when the composition is subjected to an alkaline degradation and recombination process. If the total number of moles of carbonyl bonds in the compound (II) relative to the mass of the bisphenol (I)-containing composition exceeds the above upper limit, the content of the compound (II) is high, and crystals may precipitate in the residual liquid in the furnace after distillation of isopropenylphenol, leading to significantly poor operability of withdrawing the residual liquid.

When the bisphenol (I)-containing composition contains one type of compound (II), the total number of moles of carbonyl bonds in the compound (II) relative to the mass of the bisphenol (I)-containing composition can be calculated by dividing the number of moles of carbonyl bonds calculated for the compound (II) by the mass of the bisphenol (I)-containing composition. When the bisphenol (I)-containing composition contains more than one type of compound (II), the total

number of moles of carbonyl bonds in the compound (II) relative to the mass of the bisphenol (I)-containing composition can be calculated by calculating the number of moles of carbonyl bonds in each type of the compound (II) and dividing the total of the calculated number of moles of carbonyl bond by the mass of the bisphenol (I)-containing composition.

The number of moles of carbonyl bonds (-C(=O)-) in the compound (II) can be calculated by dividing the mass of the compound (II) by the molecular weight of the compound (II) and multiplying the result by the number of carbonyl bonds in the compound (II) (that is, (mass [g] of compound (II)/molecular weight [g/μmol] of compound (II) × (number of carbonyl bonds)).

For example, when the bisphenol (I)-containing composition contains 1 μmol of the compound (II) represented by the above formula (i), the total number of moles of carbonyl bonds is 1 μmol. When the bisphenol (I)-containing composition contains 1 μmol of the compound (II) represented by the above formula (iv), the total number of moles of carbonyl bonds is 2 μmol. When the bisphenol (I)-containing composition contains 1 μmol of the compound (II) represented by the above formula (i) and 1 μmol of the compound (II) represented by the above formula (iv), the total number of moles of carbonyl bonds is 3 μmol. When the bisphenol (I)-containing composition contains 1 μmol of the compound (II) represented by the above formula (ii), 1 μmol of the compound (II) represented by the above formula (iii) and 1 μmol of the compound (II) represented by the above formula (v), the total number of moles of carbonyl bonds is 4 μmol.

The compound (II) can be detected and quantified using a standard reversed-phase column for high-speed analysis, as with the bisphenol (I). Examples of the standard reversed-phase column for high-speed analysis include an ODS-based column having an inner diameter of 3 to 4.6 mm, a length of 10 to 30 cm and a particle size of 3 to 5 μm. Specifically, Scherzo SM-C18, Cadenza CD-C18 and Unison UK-C18 manufactured by Imtakt Corporation can be used. The sizes thereof will be described in detail in the Examples.

[Method for producing bisphenol (I)-containing composition]

The bisphenol (I)-containing composition can be prepared by adding an appropriate amount of a compound (II) to a bisphenol (I) comprising bisphenol A. Alternatively, as described below, a compound (II) can be produced together with bisphenol A in a reaction system when producing bisphenol A, so that the bisphenol A product containing the compound (II) prepared is a bisphenol (I)-containing composition of the present invention.

The method for producing a bisphenol (I)-containing composition of the present invention, which contains 90% by mass or more of the bisphenol (I) comprising bisphenol A, and a predetermined proportion of the compound (II), is not particularly limited, but examples thereof include the following methods. • A method for producing a bisphenol (I)-containing composition, (1): a method for producing a bisphenol (I)-containing composition comprising: a step of adding the compound (II) to the bisphenol (I) comprising bisphenol A.

- A method for producing a bisphenol (I)-containing composition, (2): a method for producing a bisphenol (I)-containing composition comprising: a BPA production step of producing a compound (II) as a by-product when producing bisphenol A.

<Method for producing bisphenol (I)-containing composition, (1)>

The process of adding the compound (II) to the bisphenol (I) in the method for producing a bisphenol (I)-containing composition, (1) may be a process of adding the compound (II) to a solid bisphenol (I) or a process of adding the compound (II) to a molten bisphenol (I). Since the process of adding the compound (II) to the bisphenol (I) in the method for producing a bisphenol (I)-containing composition, (1) requires the compound (II) to be prepared separately, preferred is the method for producing a bisphenol (I)-containing composition, (2), and specifically, the compound (II) is preferably produced as a by-product in a reaction system for producing bisphenol A so that the compound (II) is contained at a predetermined proportion in the product.

<Method for producing bisphenol (I)-containing composition, (2)>

The method for producing a bisphenol (I)-containing composition, (2) is a method comprising a BPA production step of producing a compound (II) as a by-product when producing bisphenol A.

The BPA production step preferably comprises degrading a polycarbonate resin to produce bisphenol A while producing the compound (II) as a by-product, thereby obtaining the bisphenol (I)-containing composition comprising bisphenol A and the compound (II). As a process for degrading a polycarbonate resin, various degradation processes are known for each product derived from the carbonate ester unit of the resulting polycarbonate resin. For example, in hydrolysis with water, the product derived from the carbonate ester unit of the polycarbonate resin is obtained as carbon dioxide. Examples of the other process for degrading a polycarbonate resin include phenolysis with phenols, alcoholysis with alcohols, and aminolysis with amines. Whichever degradation process is selected, the resulting product derived from

the bisphenol unit contains bisphenol (I) as a main product.

Hereinafter, the method for producing a bisphenol (I)-containing composition, (2) will be described in detail, by way of an exemplary method in which a BPA production step comprises: PC degradation by degrading a polycarbonate resin in the presence of a solvent, to obtain a degradation liquid containing bisphenol A and a compound (II); and recovery by removing a bisphenol (I)-containing composition from the degradation liquid obtained in the PC degradation.

(PC degradation)

In the PC degradation, a polycarbonate resin can be dissolved in a solvent and degraded to produce bisphenol A. At the same time, a compound (II) can be produced as a by-product. As long as at least a portion of the polycarbonate resin is dissolved in the solvent, the degradation reaction of the polycarbonate resin proceeds. Therefore, the polycarbonate resin may be degraded in a solution having the polycarbonate resin completely dissolved in the solvent, or the polycarbonate resin may be degraded in a slurry-like solution having the polycarbonate resin dispersed in the solvent while the polycarbonate resin is dissolved.

(Polycarbonate resin (PC))

A polycarbonate resin includes a polymeric composition having carbonate ester bonds (-O-C(=O)-O-). The polycarbonate resin is sometimes simply referred to "polycarbonate", and specifically, the polycarbonate resin includes a polymer containing a structural unit derived from a bisphenol (I) represented by the formula (4).

[Chemical Formula 17]

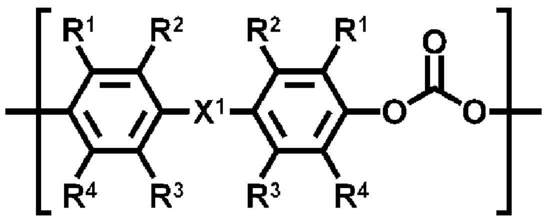

(4)

$R^1$ to $R^4$ and $X^1$ in the formula (4) have the same meanings as defined for $R^1$ to $R^4$ and $X^1$ in the above formula (1). Preferred examples and specific examples thereof are as described for the above formula (1).

The polycarbonate resin to be used in the method for producing a bisphenol (I)-containing composition, (2) of the present invention includes a bisphenol A-type polycarbonate resin having the structural unit derived from bisphenol A (the structural unit represented by the above formula (4) wherein $R^1$ to $R^4$ are hydrogen atoms, and $X^1$ is "*-$CR^{10}R^{11}$-*" wherein $R^{10}$ and $R^{11}$ are methyl groups). The bisphenol A-type polycarbonate resin may be substantially composed of the structural unit derived from bisphenol A, or may be a copolymer having the structural unit derived from bisphenol A and the structural unit derived from a bisphenol (I) other than bisphenol A. Two or more types of polycarbonate resins may be mixed and used as long as a bisphenol A-type polycarbonate resin is included, or a bisphenol A-type polycarbonate resin and another polycarbonate resin may be mixed and used.

The polycarbonate resin to be used may be a polycarbonate resin alone, or may also be a composition containing a polycarbonate resin and a resin other than the polycarbonate resin, such as a copolymer or polymer alloy of the polycarbonate resin with another resin. Examples of the composition containing a polycarbonate resin and a resin other than the polycarbonate resin include a polycarbonate/polyester copolymer, a polycarbonate/polyester alloy, a polycarbonate/polyarylate copolymer, and a polycarbonate/polyarylate alloy. When using the composition containing a polycarbonate resin and a resin other than the polycarbonate resin, a composition comprising the polycarbonate resin as a main component (a composition comprising 50% by mass or more of the polycarbonate resin) is suitable. The starting material (a polycarbonate resin or a composition comprising a polycarbonate and another resin) preferably contain 50% by mass or more of the structural unit derived from bisphenol A, and the content of the structural unit derived from bisphenol A in the starting material to be used may be 60% by mass or more, 70% by mass or more, 80% by mass or more, or 90% by mass or more.

(Solvent)

In order to dissolve the polycarbonate resin, the polycarbonate resin can be degraded in the presence of a solvent. The structure of the compound (II) produced as a by-product depends on the solvent to be used, and the solvent is therefore appropriately selected according to the structure of the compound (II).

When the bisphenol dimer is allowed to remain and used as compound (II), the solvent to be used is not particularly limited as long as it dissolves the polycarbonate resin; however, a solvent having a boiling point of 200°C or less is preferably used in consideration of recycling. Also, the solvent to be used is preferably a stable solvent that is less-reactive and does not cause undesired reactions (such as an aromatic hydrocarbon). The solvent to be used can be also a phenol or an alcohol that degrades the polycarbonate resin by a transesterification reaction.

Specifically, a bisphenol A-type polycarbonate resin can be degraded in the presence of a solvent to produce bisphenol A together with the compound (II) represented by the above formula (iii) as a by-product.

To produce, as a by-product, the compound (II) in which at least one hydroxyl group of a bisphenol is replaced with a carbonate ester group, an alcohol can be used as a solvent.

Specifically, when producing, as a by-product, the compound (II) having a carbonate ester group represented by the above formula (G1) wherein $R^{20}$ is an alkyl group or an aryl group, a polycarbonate resin can be degraded in the presence of an alcohol represented by "$R^{20}$-OH (wherein $R^{20}$ is the same as $R^{20}$ in the carbonate ester group)" to obtain the desired compound (II).

When producing, as a by-product, the compound (II) having a carbonate ester group represented by the above formula (G2) wherein $R^{21}$ is an alkyl group or an aryl group, a polycarbonate resin can be degraded in the presence of an alcohol represented by "$R^{21}$-OH (wherein $R^{21}$ is the same as $R^{21}$ in the carbonate ester group)" to obtain the desired compound (II).

For example, when the bisphenol A-type polycarbonate resin is degraded in the presence of methanol, one or more compounds (II) selected from the group consisting of the compounds represented by the above formula (i), (iii) and (iv) can be produced as a by-product together with bisphenol A.

When the bisphenol A-type polycarbonate resin is degraded in the presence of phenol, one or more compounds (II) selected from the group consisting of the compounds represented by the above formula (ii), (iii) and (v) can be produced as a by-product together with bisphenol A.

In order to produce, as a by-product, the compound (II) in which as least one hydroxyl group of a bisphenol is replaced with a carbamoyloxy group, a primary amine and/or a secondary amine can be used as a solvent. When the desired compound (II) is a compound (II) having a carbamoyloxy group represented by the above formula (G3) wherein $R^{22}$ is a hydrogen atom, an alkyl group or an aryl group and $R^{23}$ is an alkyl group or an aryl group, the polycarbonate resin can be degraded in the presence of an amine represented by "$NHR^{22}R^{23}$ (wherein $R^{22}$ and $R^{23}$ are the same as $R^{22}$ and $R^{23}$ of the carbamoyloxy group)" to produce the desired compound (II) as a by-product.

The compound (II) in which at least one hydroxyl group of a bisphenol is replaced with a carbamoyloxy group can be also produced as a by-product by degrading the polycarbonate resin in a less-reactive solvent such as an aromatic hydrocarbon as a solvent with a primary amine and/or a secondary amide as a catalyst.

Since the compound (II) preferably has a carbonate ester group, the solvent is preferably a solvent containing one or more selected from the group consisting of water, an alcohol, a dialkyl carbonate and an aromatic hydrocarbon, and more preferably a solvent containing one or more selected from the group consisting of water, an alcohol and a dialkyl carbonate. Examples of the alcohol include an aromatic alcohol such as phenol, cresol and xylenol; and an aliphatic alcohol such as methanol, ethanol, butanol and ethylene glycol. Examples of the dialkyl carbonate include dimethyl carbonate, ethyl methyl carbonate, diethyl carbonate and dibutyl carbonate. Examples of the aromatic hydrocarbon include toluene, xylene, ethylbenzene, diethylbenzene, isopropylbenzene and mesitylene. These solvents may be used singly or in combination.

If the mass ratio of the solvent to the polycarbonate resin (the mass of the solvent/the mass of the polycarbonate resin) is small, the dissolution rate of the polycarbonate resin decreases and the time required to produce bisphenol A therefore tends to be long. Accordingly, the mass ratio is preferably 0.01 or more, more preferably 0.03 or more, and even more preferably 0.05 or more. On the other hand, if the mass ratio is large, the volume of the solvent relative to that of the degradation vessel increases to decrease the amount of the polycarbonate resin that can be degraded at one time, and thus the pot efficiency tends to be poor. Therefore, the mass ratio is preferably 100 or less, more preferably 70 or less, and even more preferably 50 or less.

(Catalyst)

The degradation of a polycarbonate is preferably carried out with a catalyst. The catalyst may be any catalyst as long as it can promote the degradation of a polycarbonate resin, but it is preferably any catalyst selected from the group consisting of an alkali metal hydroxide, an alkali metal carbonate, an alkylamine and an acid.

Examples of the alkali metal hydroxide include sodium hydroxide and potassium hydroxide. Examples of the alkali metal carbonate include sodium carbonate, sodium hydrogen carbonate, potassium carbonate and potassium hydrogen carbonate. Examples of the alkylamine include a primary amine such as methylamine, ethylamine and propylamine; a secondary amine such as dimethylamine and diethylamine; and a tertiary amine such as trimethylamine. Examples of the acid include an inorganic acid such as hydrochloric acid, sulfuric acid and phosphoric acid, and an organic

acid such as a carboxylic acid and a sulfonic acid.

If the mass ratio of the catalyst to the polycarbonate resin (the mass of the catalyst/the mass of the polycarbonate resin) is small, the degradation rate of the polycarbonate resin decreases so that a long degradation time is needed, and the degradation efficiency thus tends to be poor. Therefore, the mass ratio is preferably 0.001 or more, more preferably 0.005 or more, and even more preferably 0.01 or more. On the other hand, if the mass ratio is large, the amount of an acid and a base required for neutralization tends to increase. Therefore, the mass ratio is preferably 50 or less, more preferably 20 or less, and even more preferably 10 or less.

The degradation temperature is not particularly limited. An appropriate temperature can be selected depending on the melting point and boiling point of the starting materials used in each degradation process. If the temperature is too low, there are concerns about a decrease in the solubility of the polycarbonate resin and solidification of the degradation liquid. Therefore, the degradation is preferably carried out at 10°C or more and more preferably at 20°C or more. If the temperature is too high, the solvent, starting material and catalyst may evaporate, making it difficult for the degradation to proceed. Therefore, the degradation is preferably carried out at 200°C or less and more preferably 180°C or less.

The reaction time is selected appropriately depending on the solubility of the polycarbonate resin, the reaction temperature, and others. However, when the reaction time is long, the produced bisphenol A tends to degrade. Therefore, the reaction time is preferably 30 hours or less, more preferably 25 hours or less, and even more preferably 20 hours or less. On the other hand, when the reaction time is short, the degradation reaction may not proceed sufficiently. Therefore, the reaction time is preferably 0.1 hours or more, more preferably 0.5 hours or more, and even more preferably 1 hour or more.

(Recovery)

The recovery is removing a bisphenol (I)-containing composition from the degradation liquid obtained in the PC degradation step. Since the degradation liquid obtained by the PC degradation contains bisphenol A and a compound (II), the solvent and catalyst can be removed to obtain a bisphenol (I)-containing composition that contains a bisphenol (I) comprising bisphenol A, as a main component, and the compound (II). In addition, the bisphenol (I)-containing composition is preferably purified to remove impurities, excess amount of the compound (II), and others, such that the total number of moles of carbonyl bonds in the compound (II) relative to the mass of the bisphenol (I)-containing composition becomes 0.05 μmol/g or more.

The obtained bisphenol (I)-containing composition can be recovered and purified according to any conventional method. For example, it can be recovered and purified by a convenient means such as crystallization or column chromatography. Specifically, after the polycarbonate resin is subjected to the degradation reaction, the catalyst and solvent are removed; an organic solvent is mixed with the resultant; the organic phase is washed with water or brine, and optionally further neutralized and washed with aqueous ammonium chloride solution or the like; and the washed organic phase is then cooled for crystallization.

Examples of the organic solvent that can be used include an aromatic hydrocarbon such as toluene, xylene, ethylbenzene, diethylbenzene, isopropylbenzene and mesitylene; an aliphatic hydrocarbon such as hexane, heptane, octane, nonane, decane, undecane and dodecane; and an aliphatic alcohol such as methanol, ethanol, n-propanol, i-propanol, n-butanol, i-butanol, t-butanol, n-pentanol, i-pentanol, n-hexanol, n-heptanol, n-octanol, n-nonanol, n-decanol, n-undecanol, n-dodecanol, ethylene glycol, diethylene glycol and triethylene glycol.

The starting materials, catalyst and solvent may be removed by distillation prior to crystallization. When bisphenol A is crystallized in the presence of phenol, it forms a cocrystal with phenol. Therefore, in a case where the polycarbonate resin has been degraded in phenol, it is necessary to distil off phenol prior to crystallization to prevent formation of the cocrystal.

[Method for producing bisphenol A]

The method for producing bisphenol A of the present invention comprises: an alkaline degradation step of degrading the bisphenol (I)-containing composition of the present invention in the presence of a basic catalyst to obtain a degradation liquid containing isopropenylphenol and phenol; a distillation step of distilling the degradation liquid to obtain a distillate containing isopropenylphenol and phenol; and a recombination reaction step of recombining isopropenylphenol and phenol by contacting the obtained distillate with an acidic catalyst to produce bisphenol A, thereby obtaining a solution containing bisphenol **A.**

The compound (II) can be efficiently removed from the bisphenol (I)-containing composition by using such a method for producing bisphenol A. The recovery rate of isopropenylphenol can be also improved to efficiently obtain bisphenol A not containing the compound (II). The obtained bisphenol A is suitable as a starting material for obtaining a polycarbonate resin excellent in color tone.

(Alkaline degradation step)

The bisphenol (I)-containing composition comprising bisphenol A and the compound (II) can be subjected to alkaline degradation under basic conditions to degrade bisphenol A into isopropenylphenol and phenol. Thus, a degradation liquid containing phenol and isopropenylphenol, which are degradation products of bisphenol A, can be obtained.
A basic catalyst is used for alkaline degradation of the bisphenol (I)-containing composition under basic conditions. The alkaline degradation of the bisphenol (I)-containing composition can be carried out in the presence of a basic catalyst while heating and melting the bisphenol (I)-containing composition or diluting the bisphenol (I)-containing composition with phenol.
Examples of the basic catalyst that can be used for alkaline degradation of the bisphenol (I)-containing composition include a hydroxide, an oxide, a carbonate salt and an alkoxide of an alkali metal such as sodium or potassium; and a hydroxide, an oxide, a carbonate salt and an alkoxide of an alkaline earth metal such as calcium or magnesium. Among them, sodium hydroxide or potassium hydroxide is preferred.
The alkaline degradation of the bisphenol (I)-containing composition is preferably carried out at a temperature of more than 150°C, more preferably 160°C or more, and even more preferably 170°C or more. It can be preferably carried out at 250°C or less, and more preferably 240°C or less. Too low a temperature for degrading the bisphenol (I)-containing composition is not preferred due to insufficient degradation. Too high a temperature is not preferred because it causes the undesired secondary reaction, leading to a decrease in the recovery rates of phenol and isopropenylphenol.

(Distillation step)

Phenol and isopropenylphenol in the degradation liquid can be recovered by distillation and subjected to the recombination reaction step. Distillation can be carried out at a temperature of more than 150°C. The pressure is usually 0.6 kPa to ordinary pressure, preferably 0.6 to 20 kPa, and more preferably 0.8 to 10 kPa.
The alkaline degradation step and the distillation step may be carried out sequentially or at the same time. For example, bisphenol A can be distilled while degrading it using a reaction/distillation apparatus having a reaction vessel at the bottom and a distillation column at the top. In this manner, the degradation of the bisphenol (I)-containing composition can be carried out at the same time as the distillation of phenol and isopropenylphenol.
Alternatively, the bisphenol (I)-containing composition may be degraded and the resulting solution can be transferred to the distillation column to distill the degradation liquid, thereby recovering isopropenylphenol and phenol.

(Recombination reaction step)

The phenol and isopropenylphenol obtained by distillation can be used and recombined to produce bisphenol A.
An acid is preferably used as a catalyst for recombining phenol and isopropenylphenol to obtain bisphenol A. Examples of the acidic catalyst include an inorganic acid such as sulfuric acid, hydrogen chloride and phosphoric acid; an organic acid such as p-toluenesulfonic acid, phenolsulfonic acid and methanesulfonic acid; and an acidic ion exchange resin. Among them, sulfuric acid and an acidic ion exchange resin are preferred.
The reaction temperature for recombination is usually 10 to 130°C and preferably 20 to 100°C. The contact time with the acidic catalyst is usually 5 to 200 minutes and preferably 15 120 minutes.

(Purification step)

The method for producing bisphenol A of the present invention may include a purification step of purifying bisphenol A after the recombination reaction step.
The bisphenol A produced by recombination can be purified by any conventional method. For example, it can be recovered and purified by a convenient means such as crystallization or column chromatography. Specifically, the solution after the recombination reaction step, or a solution obtained by mixing the solution after the recombination reaction step with an organic solvent, is washed with water or brine, and optionally further neutralized and washed with aqueous ammonium chloride solution or the like; and the washed organic phase is then cooled for crystallization.
Examples of the organic solvent that can be used include an aromatic hydrocarbon such as toluene, xylene, ethylbenzene, diethylbenzene, isopropylbenzene and mesitylene; an aliphatic hydrocarbon such as hexane, heptane, octane, nonane, decane, undecane and dodecane; and an aliphatic alcohol such as methanol, ethanol, n-propanol, i-propanol, n-butanol, i-butanol, t-butanol, n-pentanol, i-pentanol, n-hexanol, n-heptanol, n-octanol, n-nonanol, n-decanol, n-undecanol, n-dodecanol, ethylene glycol, diethylene glycol and triethylene glycol.
The organic solvent may be removed by distillation prior to crystallization. When bisphenol A is crystallized in the presence of phenol, it forms a cocrystal with phenol. For preventing formation of the cocrystal, it is necessary to mix the

solution after the recombination reaction step with an organic solvent, wash it, and then distill off the phenol prior to crystallization.

Alternatively, for the method for producing bisphenol A of the present invention, at least one of the steps thereof may be carried out in a BPA production plant for producing bisphenol A from acetone and phenol. The method for producing bisphenol A from acetone and phenol is described, for example, in Japanese Patent Laid-Open No. 2007-224020.

As shown in Figure 1, specific examples of the BPA production plant include a BPA production plant (P1) comprising:

a BPA synthesis means of performing dehydrative condensation of acetone (AT) and phenol (PL) in the presence of an acidic catalyst to obtain a reaction liquid c1 containing bisphenol A;
a concentration means of distilling off an unreacted acetone (AT) and water (W) from the reaction liquid c1 to obtain a concentrate c2;
a separation means of subjecting the concentrate c2 to crystallization to obtain a slurry liquid and subjecting the slurry liquid to solid-liquid separation to separate it into a mother liquid c3 and a cake containing a crystal of a phenol adduct of bisphenol A (BPA-PL);
a BPA purification means of purifying the cake to obtain bisphenol A;
a first circulation means of circulating a portion of the mother liquid c3 and feeding it to the BPA synthesis means and/or the concentration means;
a mother liquid purification means of carrying out distillation while or after degrading bisphenol A contained in the mother liquid c3 to obtain a distillate c4 containing isopropenylphenol (IPP) and phenol (PL), and contacting the obtained distillate c4 with an acidic catalyst to obtain a reaction liquid c5 containing bisphenol A; and
a second circulation means of feeding the reaction liquid c5 to the BPA synthesis means and/or the concentration means.

In the method for producing bisphenol A of the present invention, the solution containing bisphenol A obtained in the recombination reaction step may be fed to the BPA synthesis means and/or the concentration means of the BPA production plant (P1), as shown in Figure 1. In this way, the bisphenol A can be purified in the separation means and the BPA purification means together with the bisphenol A produced from acetone and phenol.

Alternatively, in the method for producing bisphenol A of the present invention, the bisphenol (I)-containing composition of the present invention may be fed to a mother liquid purification means of a BPA production plant, and the alkaline degradation step, the distillation step and the recombination reaction step may be carried out in the mother liquid purification means. The bisphenol (I)-containing composition of the present invention can be fed by heating and melting it or appropriately diluting it with phenol.

For example, as shown in Figure 2, the BPA production plant (P2) can be used that has, as a mother liquid purification means, an alkaline degradation and distillation means for degrading and distilling bisphenol A contained in the mother liquid c3 to obtain a distillate c4 containing isopropenylphenol and phenol; and a recombination reaction means for contacting the distillate c4 with an acidic catalyst to obtain a reaction liquid c5 containing bisphenol A. In the method for producing bisphenol A of the present invention, the bisphenol (I)-containing composition of the present invention may be subjected to an alkaline degradation step and a distillation step by feeding it to the alkaline degradation and distillation means of the BPA production plant (P2) by heating and melting it or appropriately diluting it with phenol, and then subjected to a recombination reaction step in a recombination reaction means ((A) in Figure 2).

Alternatively, in the method for producing bisphenol A of the present invention, the distillate obtained in the distillation step may be fed to a mother liquid purification means of a BPA production plant and subjected to the recombination reaction step in the mother liquid purification means.

For example, the distillate obtained in the distillation step may be fed to the recombination reaction means of the BPA production plant (P2) shown in Figure 2, and then subjected to the recombination reaction step in the recombination reaction means ((B) in Figure 2).

[Method for producing polycarbonate resin]

The present invention relates to a method for producing a polycarbonate resin by using the bisphenol A obtained by the method for producing bisphenol A of the present invention to produce a polycarbonate resin (hereinafter sometimes referred to as the "method for producing a polycarbonate resin of the present invention").

Specifically, it can be a method for producing a polycarbonate resin comprising: a first step of obtaining bisphenol A by the method for producing bisphenol A of the present invention; and a second step of polymerizing the resulting bisphenol compound to obtain a polycarbonate resin.

The polycarbonate resin to be obtained by the method for producing a polycarbonate resin of the present

invention can be produced, for example, by a method comprising subjecting bisphenol A including bisphenol A obtained by the method for producing bisphenol A of the present invention and a carbonate diester such as diphenyl carbonate to a transesterification reaction in the presence of an alkali metal compound and/or an alkaline earth metal compound. As the bisphenol A serving as the starting material, the bisphenol A obtained by the method for producing bisphenol A of the present invention may be used alone, or the bisphenol A obtained by the method for producing bisphenol A of the present invention may be used in combination with the bisphenol A obtained by a method other than the method for producing bisphenol A of the present invention.

The above-described transesterification reaction can be carried out by any known method selected appropriately. One example thereof in which bisphenol A and diphenyl carbonate are used as starting materials is described below.

In the above-described method for producing polycarbonate resin, diphenyl carbonate is preferably used in an excess amount over bisphenol A. The amount of diphenyl carbonate to be used is preferably large relatively to bisphenol A, in view of producing a polycarbonate resin with a small number of terminal hydroxy groups and therefore having greater thermal stability of the polymer. On the other hand, it is preferably small in view of a fast rate of transesterification reaction and ease of producing a polycarbonate resin having the desired molecular weight. Therefore, the amount of diphenyl carbonate to be used is usually 1.001 mol or more and preferably 1.002 mol or more, and usually 1.3 mol or less and preferably 1.2 mol or less, per 1 mol of bisphenol.

For the process for feeding the starting materials, bisphenol A and diphenyl carbonate can be fed in a solid state, but one or both of them are preferably melted and fed in a liquid state.

When producing a polycarbonate resin by the transesterification reaction of diphenyl carbonate and bisphenol A, a transesterification catalyst is usually used. In the above-described method for producing a polycarbonate resin, the transesterification catalyst used is preferably an alkali metal compound and/or an alkaline earth metal compound. These may be used singly or in combination of any two or more in any ratio. For practical purposes, an alkali metal compound is desirably used.

The amount of catalyst used is usually 0.05 μmol or more, preferably 0.08 μmol or more and more preferably 0.10 μmol or more, and usually 100 μmol or less, preferably 50 μmol or less and more preferably 20 μmol or less, per 1 mol of bisphenol A or diphenyl carbonate. The catalyst used in an amount within the above range facilitates obtaining the polymerization activity required to produce a polycarbonate resin having the desired molecular weight, and also facilitates obtaining a polycarbonate resin excellent in polymer color tone and excellent in flowability during shaping due to less excessive polymer branching.

To produce a polycarbonate resin by the above method, it is preferable to continuously feed both of the above starting materials to a starting material-mixing vessel, and continuously feed the resulting mixture and the transesterification catalyst to a polymerization vessel.

In the production of a polycarbonate resin by the transesterification process, usually, both starting materials fed to the starting material-mixing vessel are stirred uniformly and then fed to the polymerization vessel, to which the catalyst is added, and a polymer is thus produced.

The polycarbonate resin obtained by the method for producing a polycarbonate resin of the present invention preferably has a viscosity average molecular weight of 15,000 or more and 35,000 or less, in view of excellent flowability during shaping.

## Examples

Hereinafter, the present invention will be described more specifically by way of Examples and Comparative Example, but the present invention is not limited by the following Examples unless it does not depart from the gist thereof.

### [Starting materials and reagents]

Bisphenol A (BPA) and diphenyl carbonate used were products manufactured by Mitsubishi Chemical Corporation.

Phenol, sodium hydroxide, sulfuric acid, toluene, methanol, ethylene glycol, diethylamine, dimethyl carbonate, acetonitrile and cesium carbonate used were reagents manufactured by FUJIFILM Wako Pure Chemical Corporation.

The polycarbonate resin used was a polycarbonate resin, "NOVAREX (R) M7027BF" manufactured by Mitsubishi Chemical Engineering-Plastics Corporation.

Confirmation of the production of bisphenol A, determination of its purity, and quantification of components that were considered to be an undegraded polycarbonate resin and stabilizers (components other than phenol or bisphenol A) were carried out in the following manner using high performance liquid chromatography under the following conditions.

[Analysis of bisphenol A and compound (II)]

- Apparatus: "LC10A" manufactured by Shimadzu Corporation Unison UK C18 3 μm 250 × 4.6 mm I.D.
- Analysis temperature: 40°C
- Eluent composition: Liquid A 10 vol%/ Liquid B 90 vol% isocratic mode

  Liquid A: water
  Liquid B: acetonitrile

- Analysis time: 30 minutes
- Mass spectrometer: "Agilent LC/MS6130" manufactured by Agilent Technologies Inc.
- Ion source: ESI (Positive/Negative) AJS probe used

[Preparative isolation of compound (II)]

Preparative isolation of a compound (II) was carried out under the same conditions as those in the above [Analysis of bisphenol A and a compound (II)].

- Injection volume 400 μL per time; carried out 30 times

[Analysis and quantification of isopropenylphenol]

- Quantification method: Internal standard method involving use of biphenyl as an internal standard
- Apparatus: LC-2010A manufactured by Shimadzu Corporation, Waters Corporation 5 μm 150 mm × 4.6 mm I.D.
- Method: Low pressure gradient method
- Analysis temperature: 40°C
- Eluent composition:

  Liquid A: acetonitrile
  Liquid B: a solution of 85% phosphoric acid: water = 1 mL: 999 mL

At the analysis time of 0 minutes, the eluent composition was liquid A:liquid B= 35:65 (volume ratio; the same applies hereinafter); at the analysis time of 0 to 5 minutes, the eluent composition was liquid A:liquid B = 35:65; and then at the analysis time of 5 to 40 minutes, the eluent composition gradually changed to liquid A:liquid B = 90:10.

- Flow rate: 0.85 mL/min
- Detection wavelength: 280 nm

[Viscosity average molecular weight (Mv)]

The viscosity average molecular weight (Mv) was determined by dissolving a polycarbonate resin in methylene chloride (concentration: 6.0 g/L), measuring the specific viscosity (ηsp) thereof at 20°C with an Ubbelohde viscometer, and calculating the viscosity average molecular weight (Mv) according to the following formula.

$$\eta sp/C = [\eta](1 + 0.28\eta sp)$$

$$[\eta] = 1.23 \times 10^{-4} Mv^{0.83}$$

[Reference Example]

Ten grams of BPA, 1 g of diphenyl carbonate and 50 μL of a 0.1% by weight aqueous sodium hydroxide solution were weighed into a PTFE test tube, and the test tube was sealed with a PTFE stopper and then was heated to 190°C with

an aluminum block heater to allow the mixture to react for 2 hours. After 2 hours, the liquid in the test tube was sampled and analyzed, and as a result, three peaks were detected in addition to peaks corresponding to phenol, BPA and diphenyl carbonate. The three peaks corresponded to MW 348, MW 482 and MW 468, respectively, and it was thus confirmed that produced were (4-(2-(4-hydroxyphenyl)isopropyl)phenyl)phenyl carbonate (hereinafter referred to as "PB1"), bis(4-(2-(4-hydroxyphenyl)isopropyl)phenyl)carbonate (hereinafter referred to as "PB2"), and diphenyl(propane-2,2-diylbis(4,1-phenylene))bis(carbonate) (hereinafter referred to as "PB3"), which were products of polycondensation of BPA and diphenyl carbonate.

PB1, PB2, and PB3 were subjected to preparative isolation using liquid chromatography to obtain 1.08 g of PB1, 0.60 g of PB2, and 0.15 g of PB3.

The structure of each of PB1, PB2 and PB3 is as follows, and these compounds correspond to the compound (II).

[Chemical Formula 18]

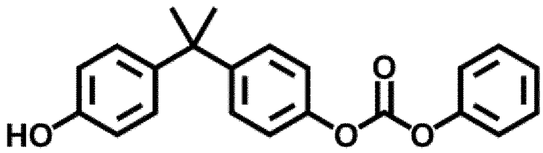

(PB1)

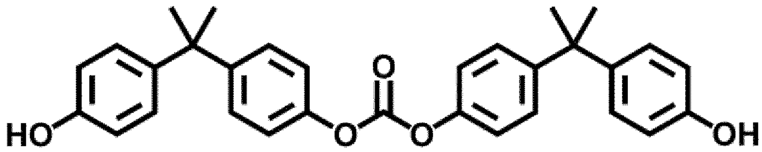

(PB2)

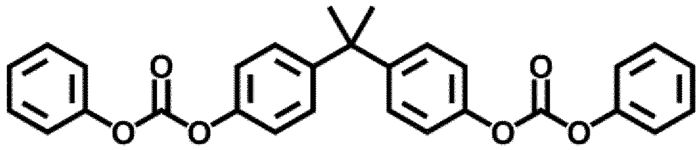

(PB3)

[Example 1]

BPA manufactured by Mitsubishi Chemical Corporation and PB1 obtained in Reference Example 1 were mixed to prepare a bisphenol (I)-containing composition a, which contained 20 wtppm of PB1 (in terms of carbonyl bond units, containing 0.06 μmol per gram of the bisphenol (I)-containing composition a).

The composition analysis of BPA manufactured by Mitsubishi Chemical Corporation showed that the purity (content of bisphenol A) was 99.9% by weight (% by mass) or more, and that the content of the compound corresponding to the compound (II) was below the detection limit.

Forty grams of phenol, 150 g (0.658 mol) of the bisphenol (I)-containing composition a, and 1.2 g of a 25% by weight aqueous sodium hydroxide solution were weighed in a nitrogen atmosphere into a jacketed separable flask equipped with a stirring blade, a thermometer, a Dimroth condenser, a distillation tube, a distillate receptable and a pressure regulator, and the temperature was gradually increased until the internal temperature reached 200°C. After the temperature reached 200°C, the pressure was gradually reduced to 10 kPa over 30 minutes to distill out phenol and isopropenylphenol. When 80 minutes elapsed, no more distillation was seen, and the pressure was restored. The mass of the distillate was 135 g, and the composition analysis showed that the distillate contained 29.6% by weight of isopropenylphenol (135 g × 29.6% by weight / 100 = 40 g). The recovery rate of the isopropenylphenol based on BPA fed to the reaction was 45.4 mol% (40 g ÷ MW 134 ÷ 0.658 mol × 100 = 45.4 mol%).

The evaporation residue remaining in the separable flask was in the form of a homogeneous liquid.

[Example 2]

BPA manufactured by Mitsubishi Chemical Corporation and PB1 obtained in Reference Example 1 were mixed to

prepare a bisphenol (I)-containing composition b, which contained 200 wtppm of PB1 (in terms of carbonyl bond units, containing 0.57 μmol per gram of the bisphenol (I)-containing composition b).

155 g of a distillate was obtained by the operation carried out in the same manner as in Example 1, except that the bisphenol (I)-containing composition a was changed to the bisphenol (I)-containing composition b. The composition analysis showed that the distillate contained 31.6% by weight of isopropenylphenol (155 g × 31.6% by weight / 100 = 49 g). The recovery rate of the isopropenylphenol based on BPA fed to the reaction was 55.6 mol% ((49 g / MW 134) / 0.658 mol × 100 = 55.6 mol%).

The evaporation residue remaining in the separable flask was in the form of a homogeneous liquid.

[Example 3]

BPA manufactured by Mitsubishi Chemical Corporation and PB1 and PB2 obtained in Reference Example 1 were mixed to prepare a bisphenol (I)-containing composition c, which contained 10 wtppm of PB1 and 500 wtppm of PB2 (in terms of carbonyl bond units, containing 1.07 μmol per gram of the bisphenol (I)-containing composition c).

156 g of a distillate was obtained by the operation carried out in the same manner as in Example 1, except that the bisphenol (I)-containing composition a was changed to the bisphenol (I)-containing composition c. The composition analysis showed that the distillate contained 31.4% by weight of isopropenylphenol (156 g × 31.4% by weight / 100 = 49 g). The recovery rate of the isopropenylphenol based on BPA fed to the reaction was 55.6 mol% ((49 g / MW 134) / 0.658 mol × 100 = 55.6 mol%).

The evaporation residue remaining in the separable flask was in the form of a homogeneous liquid.

[Example 4]

In a jacketed separable flask equipped with a Dimroth condenser, a stirring blade and a thermometer were placed 200 g of a polycarbonate resin (the mass of the structural unit (repeating unit) of the polycarbonate resin was 254 g/mol, and therefore, 80 g / 254 g/mol = 0.787 mol), 147 g of water, 600 g of phenol, and 23.5 g of a 25% by weight aqueous sodium hydroxide solution at room temperature in a nitrogen atmosphere. The reaction liquid was in the form of a slurry.

Thereafter, the internal temperature was increased to 90°C, and the reaction liquid was allowed to react for 4 hours while keeping the internal temperature at 90°C to obtain a homogeneous solution.

A 70% by weight aqueous sulfuric acid solution was fed to the obtained reaction liquid until the aqueous phase reached a pH of 7.5, and as a result, carbon dioxide gas was generated. Thereafter, stirring was stopped, the mixture was subjected to oil-water separation, and the aqueous phase was drained from the flask to obtain 980 g of an organic phase 1.

A portion of the obtained organic phase 1 was subjected to the composition analysis by high performance liquid chromatography to confirm the production of bisphenol A.

The obtained organic phase 1 was transferred to a distillation apparatus equipped with a thermometer, a stirring blade, a distillation tube and a pressure regulator. The internal temperature was gradually increased to 130°C while monitoring the amount of distillate. The internal pressure was gradually reduced from ordinary pressure to 10 kPa, and water and phenol were distilled off until the distillate rate (distillate / organic phase 1 × 100) was 75% by weight.

Thereafter, the internal pressure of the flask was restored with nitrogen, the internal temperature was decreased to 80°C, and 800 g of toluene and 100 g of water were added to the flask to obtain an organic phase 2. The temperature of the obtained organic phase 2 was decreased to 10°C to obtain a slurry. The obtained slurry was filtered to obtain 250 g of a crude cake.

The obtained crude cake was fed again in a nitrogen atmosphere to a jacketed separable flask equipped with a Dimroth condenser, a stirring blade and a thermometer, and dissolved by adding 1,200 g of toluene and increasing the temperature to 80°C with stirring, to obtain an organic phase 3.

To the organic phase 3, 200 g of water was added, and the resultant was stirred for 15 minutes to wash the organic phase 3. After 15 minutes, stirring was stopped, the organic phase 3 was subjected to a two-phase separation into an organic phase and a water phase, and the water phase was removed to obtain an organic phase 4. This operation was repeated three times to obtain the organic phase 4.

The temperature of the obtained organic phase 4 was decreased to 10°C to obtain a slurry. The obtained slurry was filtered to obtain 220 g of a cake.

The obtained cake was dried with a rotary evaporator to obtain 175 g of a bisphenol (I)-containing composition d. The composition analysis of the bisphenol (I)-containing composition d was carried out to confirm that the purity was 99.9% by weight or more, and that it contained 40 wtppm of PB1, 400 wtppm of PB2 and 220 wtppm of PB3 (in terms of carbonyl bond units, containing 1.88 μmol per gram of the bisphenol (I)-containing composition).

155 g of a distillate was obtained by the operation carried out in the same manner as in Example 1, except that the bisphenol (I)-containing composition a was changed to the bisphenol (I)-containing composition d. The composition analysis showed that the distillate contained 31.0% by weight of isopropenylphenol (155 g × 31.0% by weight / 100 = 48 g).

The recovery rate of the isopropenylphenol based on BPA fed to the reaction was 54.4 mol% ((48 g / MW 134) / 0.658 mol × 100 = 54.4 mol%).

The evaporation residue remaining in the separable flask was in the form of a homogeneous liquid.

[Example 5]

A bisphenol (I)-containing composition e was obtained by the operation carried out in the same manner as in Example 4, except that the degradation temperature of a polycarbonate resin was changed from 90°C to 80°C, and the bisphenol (I)-containing composition e had a purity of 99.9% by weight or more and contained 5 wtppm of PB1, 4,210 wtppm of PB2 and 340 wtppm of PB3 (in terms of carbonyl bond units, containing 10.20 μmol per gram of the bisphenol (I)-containing composition e).

158 g of a distillate was obtained by the operation carried out in the same manner as in Example 1, except that the bisphenol (I)-containing composition a was changed to the bisphenol (I)-containing composition e. The composition analysis showed that the distillate contained 31.6% by weight of isopropenylphenol (158 g × 31.6% by weight / 100 = 50 g). The recovery rate of the isopropenylphenol based on BPA fed to the reaction was 56.7 mol% ((50 g / MW 134) / 0.658 mol × 100 = 56.7 mol%).

[Example 6]

A bisphenol (I)-containing composition f was obtained by the operation carried out in the same manner as in Example 4, except that the degradation temperature of a polycarbonate resin was changed from 90°C to 70°C and that the amount of a 25% by weight aqueous sodium hydroxide solution fed was changed from 23.5 g to 10.0 g, and the bisphenol (I)-containing composition f had a purity of 98.9% by weight and contained 700 wtppm of PB1, 9,500 wtppm of PB2 and 1,220 wtppm of PB3 (in terms of carbonyl bond units, containing 26.93 μmol per gram of the bisphenol (I)-containing composition e).

160 g of a distillate was obtained by the operation carried out in the same manner as in Example 1, except that the bisphenol (I)-containing composition a was changed to the bisphenol (I)-containing composition f. The composition analysis showed that the distillate contained 32.5% by weight of isopropenylphenol (160 g × 32.5% by weight / 100 = 52 g). The recovery rate of the isopropenylphenol based on BPA fed to the reaction was 59.0 mol% ((52 g / MW 134) / 0.658 mol × 100 = 59.0 mol%).

A solid precipitated in the evaporation residue remaining in the separable flask, and it was difficult to withdraw.

[Comparative Example 1]

120 g of a distillate was obtained by the operation carried out in the same manner as in Example 1, except that the bisphenol (I)-containing composition a was changed to BPA manufactured by Mitsubishi Chemical Corporation. The composition analysis showed that the distillate contained 20.8% by weight of isopropenylphenol (120 g × 20.8% by weight / 100 = 25 g). The recovery rate of the isopropenylphenol based on BPA fed to the reaction was 28.4 mol% ((25 g / MW 134) / 0.658 mol × 100 = 28.4 mol%).

The evaporation residue remaining in the separable flask was in the form of a homogeneous liquid.

Table 1 summarizes the results of Examples 1 to 6 and Comparative Example 1.

As can be seen from Table 1, the recovery rate of isopropenylphenol was improved by using, as a starting material, a bisphenol (I)-containing composition containing a specific amount of a compound corresponding to compound (II). However, if it was contained in an excessive amount, a solid precipitates in the evaporation residue after distilling off isopropenylphenol, making the withdrawal thereof difficult, and workability tended to be reduced.

[Table 1]

| | Carbonyl bond units in bisphenol (I)-containing composition μmol/g | IPP recovery rate mol% | State of evaporation residue |
|---|---|---|---|
| **Example 1** | **0.06** | **45.4** | **Homogeneous** |
| **Example 2** | **0.57** | **55.6** | **Homogeneous** |
| **Example 3** | **1.07** | **55.6** | **Homogeneous** |
| **Example 4** | **1.88** | **54.4** | **Homogeneous** |
| **Example 5** | **10.20** | **56.7** | **Homogeneous** |
| **Example 6** | **26.93** | **59.0** | Solid precipitated |

(continued)

| | Carbonyl bond units in bisphenol (I)-containing composition μmol/g | IPP recovery rate mol% | State of evaporation residue |
|---|---|---|---|
| Comparative Example 1 | **0.00** | **28.4** | **Homogeneous** |

[Example 7]

A 158 g portion of the distillate obtained in Example 5 (containing 50 g of isopropenylphenol) and 50 g of toluene were fed under a nitrogen gas flow to a jacketed separable flask equipped with a Dimroth condenser, a stirring blade and a thermometer, and heated to 40°C with stirring. A 10 g portion of a 10 wt% aqueous sulfuric acid solution was added thereto, and the resultant was allowed to react for 30 minutes. The reaction liquid was subjected to composition analysis to confirm that bisphenol A had been produced.

A 200 g portion of toluene and 100 g of water were added to the reaction liquid, and the temperature of the resultant was increased to 75°C with stirring. After confirming that the temperature had reached 75°C, the mixture was allowed to stand to separate into an organic phase and an aqueous phase, and the aqueous phase was removed. Thereafter, the residue was gradually cooled to 10°C to obtain a slurry liquid. The obtained slurry liquid was filtered with a vacuum filter to obtain 50 g of a crude BPA cake.

A 50 g portion of the obtained BPA cake, 250 g of toluene and 100 g of a 5 wt% aqueous sodium hydrogen carbonate solution were fed under a nitrogen gas flow to a jacketed separable flask equipped with a Dimroth condenser, a stirring blade and a thermometer, and heated to 80°C with stirring. After confirming that the temperature had reached 80°C, the mixture was allowed to stand to separate into an organic phase and an aqueous phase, and the aqueous phase was removed. To the organic phase left after the water phase was removed, 100 g of water was added, and the resultant was stirred for 20 minutes. Thereafter, it was allowed to stand to separate into an organic phase and an aqueous phase, and the water phase was removed. This operation was repeated twice. Thereafter, the residue was gradually cooled to 10°C to obtain a slurry liquid. The obtained slurry liquid was filtered with a vacuum filter to obtain 45 g of a purified cake of BPA.

The obtained purified cake was dried with a rotary evaporator to obtain 35 g of bisphenol A.

The composition analysis of the obtained bisphenol A showed that the purity was 99.9% by weight or more, and that the content of the compound corresponding to the compound (II) was below the detection limit.

[Example 8]

A 10.00 g portion (0.04 mol) of bisphenol A obtained in Example 7, 9.95 g (0.05 mol) of diphenyl carbonate and 18 μL of a 400 ppm by mass aqueous cesium carbonate solution were placed in a 45 mL glass reaction vessel equipped with a stirrer and a distillation tube. The operation of reducing the pressure in the glass reaction vessel to about 100 Pa and then restoring the pressure to atmospheric pressure with nitrogen was repeated three times to replace gas inside the reaction vessel with nitrogen. Thereafter, the reaction vessel was immersed in an oil bath at 220°C to dissolve the contents thereof.

The rotation speed of the stirrer was set to 100 rotations per minute, and the absolute pressure in the reaction vessel was reduced from 101.3 kPa to 13.3 kPa over 40 minutes while distilling off phenol produced as a by-product of the oligomerization reaction of bisphenol A and diphenyl carbonate in the reaction vessel.

Subsequently, the pressure in the reaction vessel was maintained at 13.3 kPa, and the transesterification reaction was carried out for 80 minutes while further distilling off the phenol.

Thereafter, the external temperature of the reaction vessel was increased to 290°C while the absolute pressure in the reaction vessel was reduced from 13.3 kPa to 399 Pa over 40 minutes to remove the distilled phenol outside the system.

Thereafter, the absolute pressure of the reaction vessel was reduced to 30 Pa and the polycondensation reaction was carried out. When the stirrer in the reaction vessel reached a predetermined stirring power, the polycondensation reaction was terminated. The time from increasing the temperature to 290°C to terminating the polymerization was 120 minutes.

The pressure in the reaction vessel was then restored to an absolute pressure of 101.3 kPa with nitrogen. Thereafter, the gauge pressure thereof was increased to 0.2 MPa, and a polycarbonate resin was obtained by withdrawing it from the reaction vessel. The viscosity average molecular weight (Mv) of the obtained polycarbonate resin was 26,300.

## Claims

**1.** A bisphenol (I)-containing composition comprising: a bisphenol (I) comprising bisphenol A; and a compound (II)

having a structure in which at least one hydroxyl group of a bisphenol is replaced with a carbonate ester group or a carbamoyloxy group; wherein:

the content of the bisphenol (I) in the bisphenol (I)-containing composition is 90% by mass or more; and
the total number of moles of carbonyl bonds in the compound (II) relative to the mass of the bisphenol (I)-containing composition is 0.05 μmol/g or more.

**2.** The bisphenol (I)-containing composition according to claim 1, wherein the total number of moles of carbonyl bonds in the compound (II) relative to the mass of the bisphenol (I)-containing composition is 50 μmol/g or less.

**3.** The bisphenol (I)-containing composition according to claim 1 or 2, wherein the compound (II) is a compound represented by the following formula (2) and/or formula (3):

[Chemical Formula 1]

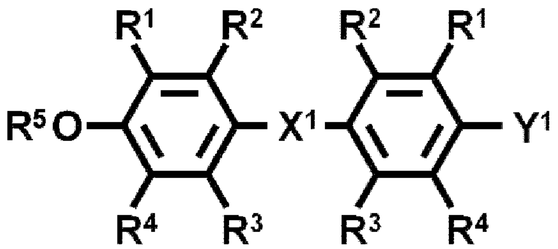

(2)

wherein

$R^1$ to $R^4$ are each independently a hydrogen atom, a halogen atom, an alkyl group, an aryl group or an alkoxy group;
$X^1$ is *-$CR^{10}R^{11}$-* (wherein * represents the bonding position to the benzene ring, and $R^{10}$ and $R^{11}$ are each independently a hydrogen atom, a halogen atom, an alkyl group, an aryl group or an alkoxy group), a cycloalkylidene group, a fluorenylidene group, a xanthenylidene group or a thioxanthenylidene group;
$R^5$ is a hydrogen atom, an alkyl group or an aryl group; and
$Y^1$ is a carbonate ester group or a carbamoyloxy group;

[Chemical Formula 2]

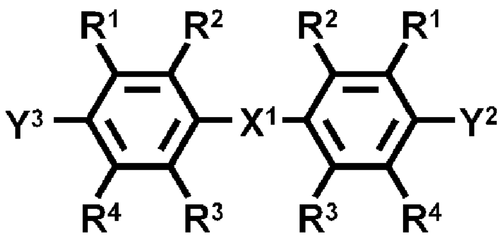

(3)

wherein
$R^1$ to $R^4$ and $X^1$ have the same meanings as defined for $R^1$ to $R^4$ and $X^1$ in the formula (2); and
$Y^2$ and $Y^3$ are each independently a carbonate ester group or a carbamoyloxy group.

**4.** The bisphenol (I)-containing composition according to claim 3, wherein the carbonate ester group is a group represented by the following formula (G1) or formula (G2) :

[Chemical Formula 3]

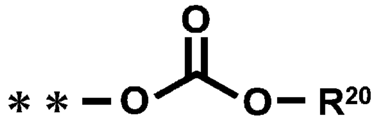

(G 1)

wherein

** represents the bonding position to the benzene ring of the bisphenol skeleton; and
$R^{20}$ is a hydrogen atom, an alkyl group or an aryl group;

[Chemical Formula 4]

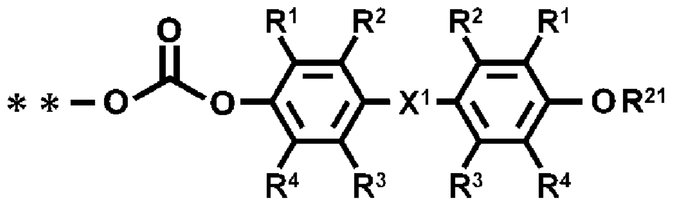

(G 2)

wherein
** represents the bonding position to the benzene ring of the bisphenol skeleton;
$R^1$ to $R^4$ are each independently a hydrogen atom, a halogen atom, an alkyl group, an aryl group or an alkoxy group;
$X^1$ is *-$CR^{10}R^{11}$-* (wherein * represents the bonding position to the benzene ring, and $R^{10}$ and $R^{11}$ are each independently a hydrogen atom, a halogen atom, an alkyl group, an aryl group or an alkoxy group), a cycloalkylidene group, a fluorenylidene group, a xanthenylidene group or a thioxanthenylidene group; and
$R^{21}$ is a hydrogen atom, an alkyl group or an aryl group.

**5.** The bisphenol (I)-containing composition according to claim 1 or 2, wherein the content of bisphenol A in the bisphenol (I) is 90% by mass or more.

**6.** The bisphenol (I)-containing composition according to claim 1 or 2, wherein the compound (II) is one or more selected from the group consisting of compounds represented by the following formulas (i) to (v).

[Chemical Formula 5]

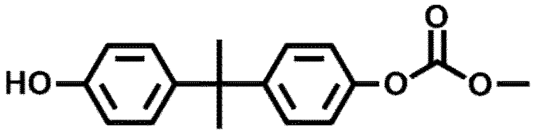

( i )

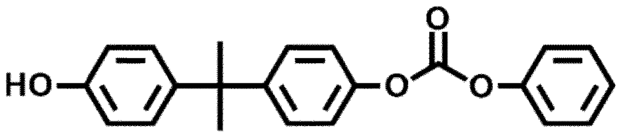

( ii )

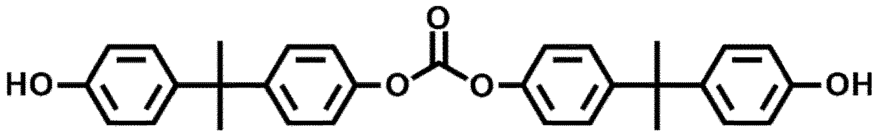

( iii )

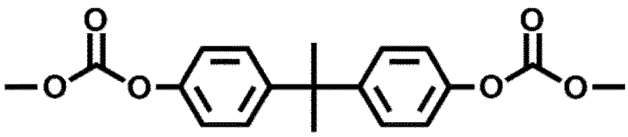

( iv )

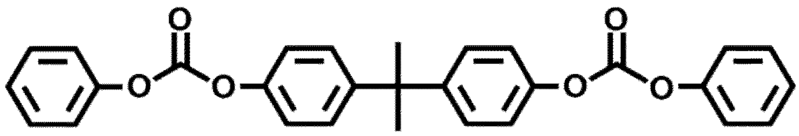

( v )

7. A method for producing the bisphenol (I)-containing composition according to claim 1 or 2, comprising: a BPA production step of producing the compound (II) as a by-product when producing bisphenol A.

8. The method for producing a bisphenol (I)-containing composition according to claim 7, wherein the BPA production step comprises degrading a polycarbonate resin to produce bisphenol A while producing the compound (II) as a by-product, thereby obtaining the bisphenol (I)-containing composition comprising bisphenol A and the compound (II).

9. A method for producing the bisphenol (I)-containing composition according to claim 1 or 2, comprising: a step of adding the compound (II) to the bisphenol (I) comprising bisphenol A.

10. A method for producing bisphenol A, comprising:

an alkaline degradation step of degrading the bisphenol (I)-containing composition according to claim 1 or 2 in the presence of a basic catalyst to obtain a degradation liquid containing isopropenylphenol and phenol;
a distillation step of distilling the degradation liquid to obtain a distillate containing isopropenylphenol and phenol; and
a recombination reaction step of recombining isopropenylphenol and phenol by contacting the obtained distillate with an acidic catalyst to produce bisphenol A, thereby obtaining a solution containing bisphenol A.

11. The method for producing bisphenol A according to claim 10, comprising: a step of feeding the solution containing bisphenol A obtained in the recombination reaction step to a BPA synthesis means and/or a concentration means of a BPA production plant, wherein the BPA production plant comprises:

the BPA synthesis means of performing dehydrative condensation of acetone and phenol in the presence of an acidic catalyst to obtain a reaction liquid c1 containing bisphenol A;
the concentration means of distilling off an unreacted acetone and water from the reaction liquid c1 to obtain a concentrate c2;
a separation means of subjecting the concentrate c2 to crystallization to obtain a slurry liquid and subjecting the slurry liquid to solid-liquid separation to separate it into a mother liquid c3 and a cake containing a crystal of a phenol adduct of bisphenol A;
a BPA purification means of purifying the cake to obtain bisphenol A;
a first circulation means of circulating a portion of the mother liquid c3 and feeding it to the BPA synthesis means and/or the concentration means;
a mother liquid purification means of carrying out distillation while or after degrading bisphenol A contained in the mother liquid c3 to obtain a distillate c4 containing isopropenylphenol and phenol, and contacting the obtained distillate c4 with an acidic catalyst to obtain a reaction liquid c5 containing bisphenol A; and
a second circulation means of feeding the reaction liquid c5 to the BPA synthesis means and/or the concentration means.

**12.** The method for producing bisphenol A according to claim 10, wherein:
the bisphenol (I)-containing composition is fed to a mother liquid purification means of a BPA production plant, wherein the BPA production plant comprises:

a BPA synthesis means of performing dehydrative condensation of acetone and phenol in the presence of an acidic catalyst to obtain a reaction liquid c1 containing bisphenol A;
a concentration means of distilling off an unreacted acetone and water from the reaction liquid c1 to obtain a concentrate c2;
a separation means of subjecting the concentrate c2 to crystallization to obtain a slurry liquid and subjecting the slurry liquid to solid-liquid separation to separate it into a mother liquid c3 and a cake containing a crystal of a phenol adduct of bisphenol A;
a BPA purification means of purifying the cake to obtain bisphenol A;
a first circulation means of circulating a portion of the mother liquid c3 and feeding it to the BPA synthesis means and/or the concentration means;
the mother liquid purification means of carrying out distillation while or after degrading bisphenol A contained in the mother liquid c3 to obtain a distillate c4 containing isopropenylphenol and phenol, and contacting the obtained distillate c4 with an acidic catalyst to obtain a reaction liquid c5 containing bisphenol A; and
a second circulation means of feeding the reaction liquid c5 to the BPA synthesis means and/or the concentration means; and
in the mother liquid purification means, the alkaline degradation step, the distillation step and the recombination reaction step are carried out.

**13.** The method for producing bisphenol A according to claim 10, wherein:
the distillate obtained in the distillation step is fed to a mother liquid purification means of a BPA production plant, wherein the BPA production plant comprises:

a BPA synthesis means of performing dehydrative condensation of acetone and phenol in the presence of an acidic catalyst to obtain a reaction liquid c1 containing bisphenol A;
a concentration means of distilling off an unreacted acetone and water from the reaction liquid c1 to obtain a concentrate c2;
a separation means of subjecting the concentrate c2 to crystallization to obtain a slurry liquid and subjecting the slurry liquid to solid-liquid separation to separate it into a mother liquid c3 and a cake containing a crystal of a phenol adduct of bisphenol A;
a BPA purification means of purifying the cake to obtain bisphenol A;
a first circulation means of circulating a portion of the mother liquid c3 and feeding it to the BPA synthesis means and/or the concentration means;
the mother liquid purification means of carrying out distillation while or after degrading bisphenol A contained in the mother liquid c3 to obtain a distillate c4 containing isopropenylphenol and phenol, and contacting the obtained distillate c4 with an acidic catalyst to obtain a reaction liquid c5 containing bisphenol A; and
a second circulation means of feeding the reaction liquid c5 to the BPA synthesis means and/or the concentration means; and
in the mother liquid purification means, the recombination reaction step is carried out.

**14.** A method for producing a polycarbonate resin comprising:

a first step of obtaining bisphenol A by the method for producing bisphenol A according to claim 10; and
a second step of polymerizing bisphenol A obtained in the first step to obtain a polycarbonate resin.

**15.** The method for producing a polycarbonate resin according to claim 14, wherein the viscosity average molecular weight of the polycarbonate resin is 15,000 or more and 35,000 or less.

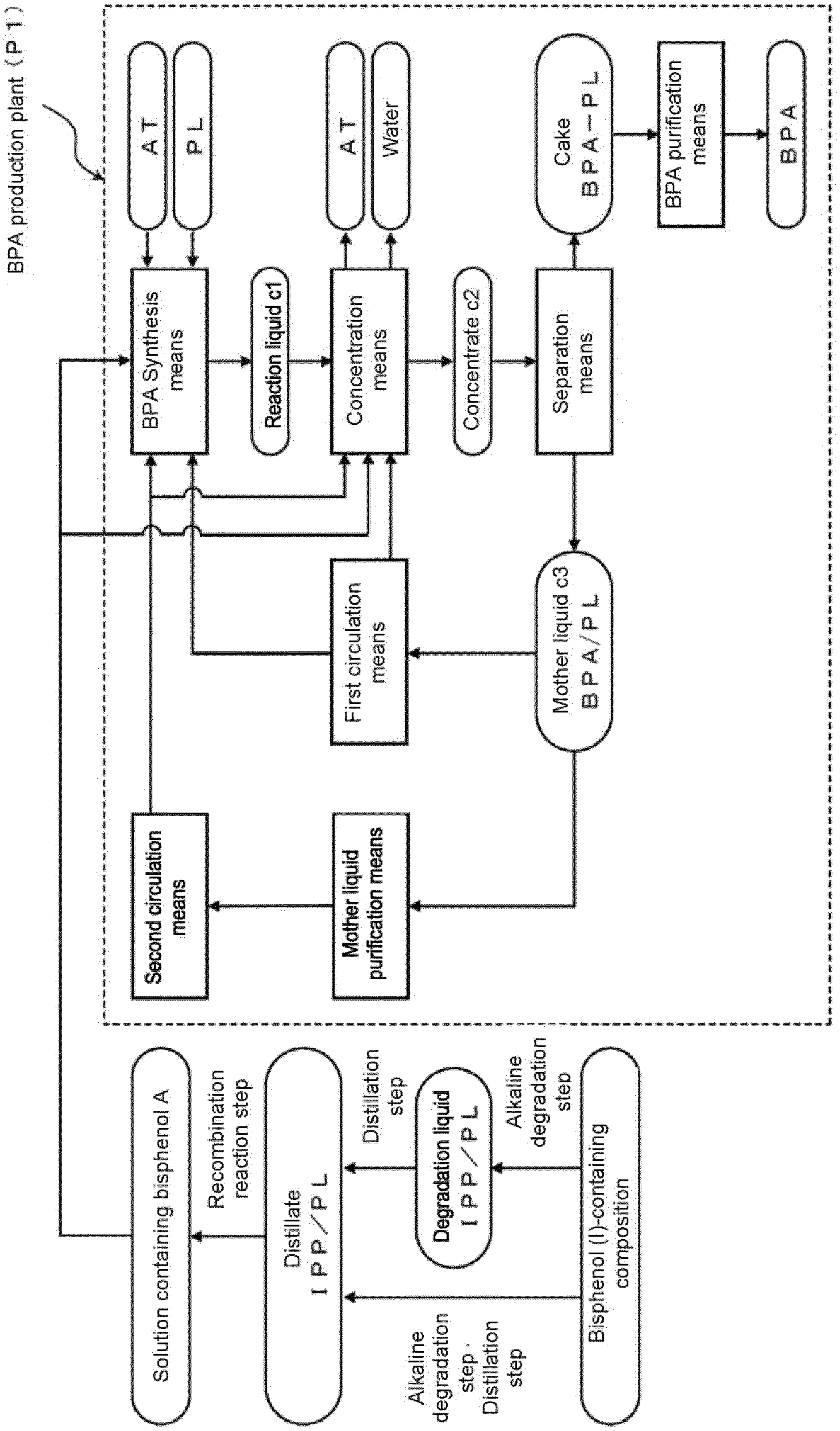
BPA production plant (P 1)
A T
P L
BPA Synthesis means
Reaction liquid c1
Concentration means
A T
Water
Concentrate c2
Separation means
Cake
B P A − P L
BPA purification means
B P A
First circulation means
Mother liquid c3
B P A / P L
Second circulation means
Mother liquid purification means
Solution containing bisphenol A
Recombination reaction step
Distillate
I P P / P L
Distillation step
Degradation liquid
I P P / P L
Alkaline degradation step
Bisphenol (I)-containing composition
Alkaline degradation step · Distillation step

Fig. 1

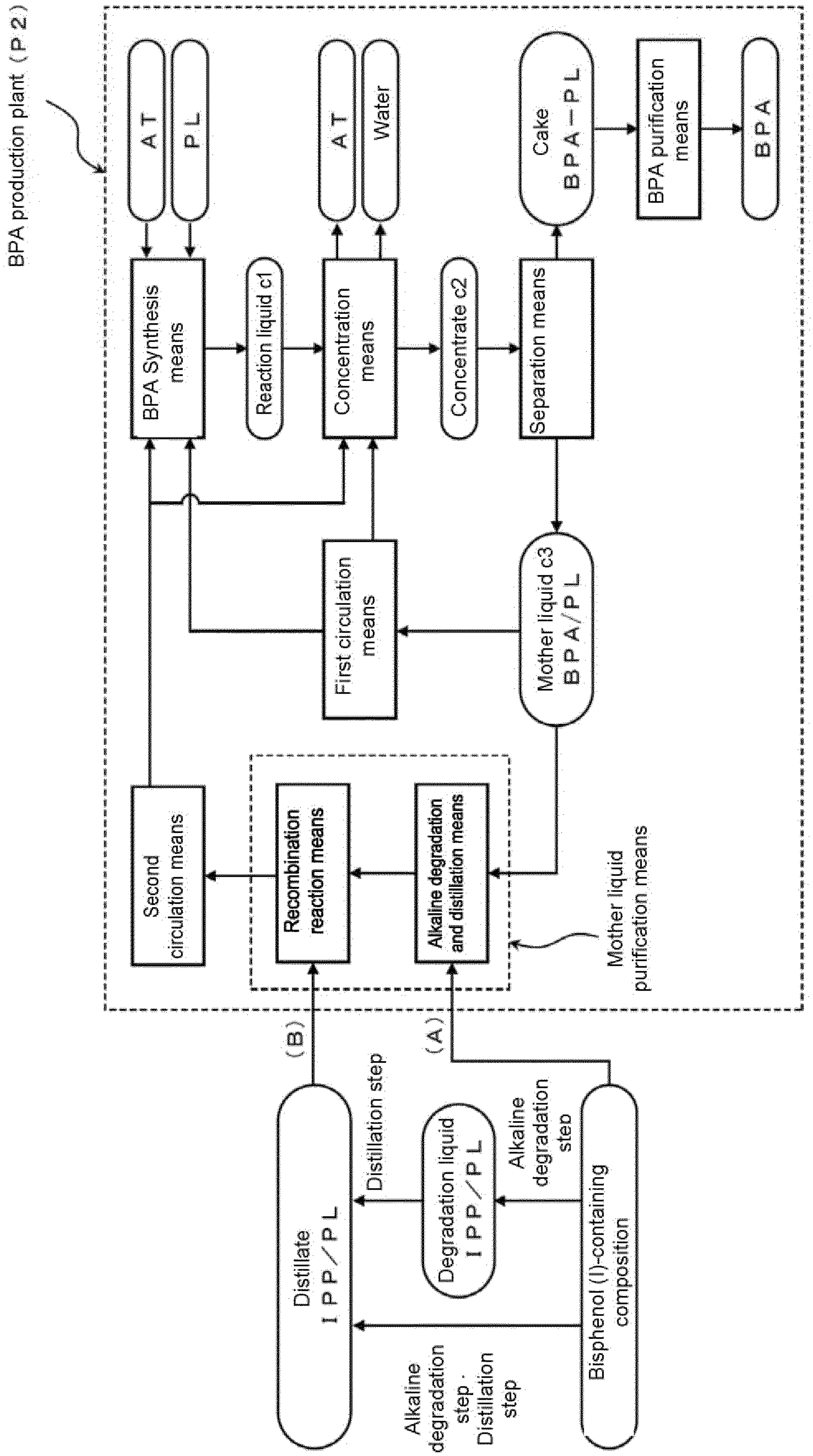
BPA production plant (P2)
AT
PL
BPA Synthesis means
Reaction liquid c1
Concentration means
AT
Water
Concentrate c2
Separation means
Cake BPA−PL
BPA purification means
BPA
First circulation means
Mother liquid c3 BPA/PL
Second circulation means
Recombination reaction means
Alkaline degradation and distillation means
Mother liquid purification means
(B)
(A)
Distillation step
Alkaline degradation step
Distillate IPP/PL
Degradation liquid IPP/PL
Bisphenol (I)-containing composition
Alkaline degradation step · Distillation step

Fig. 2

**INTERNATIONAL SEARCH REPORT**

International application No. **PCT/JP2023/016061**

**A. CLASSIFICATION OF SUBJECT MATTER**

***C07C 39/16***(2006.01)i; ***C07C 37/20***(2006.01)i; ***C07C 37/74***(2006.01)i; ***C07C 37/84***(2006.01)i; ***C07C 69/96***(2006.01)i; ***C08G 64/30***(2006.01)i

FI: C07C39/16; C07C37/20; C07C37/84; C07C37/74; C08G64/30; C07C69/96 Z

According to International Patent Classification (IPC) or to both national classification and IPC

**B. FIELDS SEARCHED**

Minimum documentation searched (classification system followed by classification symbols)

C07C39/16; C07C37/20; C07C37/74; C07C37/84; C07C69/96; C08G64/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | KR 2020-0014004 A (INDUSTRIAL COOPERATION FOUNDATION CHONBUK NATIONAL UNIVERSITY) 10 February 2020 (2020-02-10) claims, examples, test examples, table 1, in particular, experiments 8, 11, 12 | 1-8 |
| Y | | 1-8, 10-15 |
| A | | 9 |
| Y | JP 2009-242316 A (MITSUBISHI CHEMICALS CORP) 22 October 2009 (2009-10-22) claims, paragraphs [0013]-[0052] | 1-8, 10-15 |
| A | | 9 |
| A | WO 2018/134734 A1 (SABIC GLOBAL TECHNOLOGIES B. V.) 26 July 2018 (2018-07-26) claims, paragraphs [0021]-[0024], [0044]-[0049], [0060], fig. 1-7 | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 June 2023** | **27 June 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/016061**

| Patent document cited in search report | Publication date (day/month/year) | Patent family member(s) | Publication date (day/month/year) |
|---|---|---|---|
| KR 2020-0014004 A | 10 February 2020 | (Family: none) | |
| JP 2009-242316 A | 22 October 2009 | (Family: none) | |
| WO 2018/134734 A1 | 26 July 2018 | US 2021/0292477 A1<br>EP 3571240 A1<br>CN 110072910 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 62138443 A
- JP 2014040376 A
- JP 49048319 A
- JP 1230538 A
- JP 2007224020 A